# FASCICULE DE BREVET EUROPEEN

(11) **EP 0 781 829 B1**
(45) Date de publication et mention de la délivrance du brevet: **14.11.2001**
(21) Numéro de dépôt: 96402912.8
(22) Date de dépôt: 27.12.1996
(51) Int. Cl.: C10G 45/40

(54) **Procédé et dispositif d'hydrogénation sélective par distillation catalytique**
Verfahren und Vorrichtung zur selektiven Hydrierung durch katalytische Distillation
Process and apparatus for the selective hydrogenation by catalytic distillation

(30) Priorité: 27.12.1995 FR 9515530
(43) Date de publication de la demande: 02.07.1997
(73) Titulaire: INSTITUT FRANCAIS DU PETROLE, 92502 Rueil-Malmaison (FR)
(72) Inventeur: Travers, Christine, 92500 Rueil Malmaison (FR); Cosyns, Jean, 78580 Maule (FR); Cameron, Charles, 75005 Paris (FR); Nocca, Jean-Luc, 92500 Rueil Malmaison (FR); Montecot, Francoise, 78340 Les Clayes Sous Bois (FR); Viltard, Jean-Charles, 26000 Valence (FR); Dorbon, Michel, 69002 Lyon (FR); Didillon, Blaise, 92500 Rueil Malmaison (FR)

(56) Documents cités:
- EP-A- 0 552 069
- EP-A- 0 552 070
- US-A- 4 847 430
- US-A- 5 368 691

## Description

L'invention concerne un dispositif de distillation réactive comportant une zone de distillation, associée à une zone réactionnelle au moins en partie interne à ladite zone de distillation et comprenant au moins un lit catalytique, dans laquelle on réalise la transformation de la charge, en présence d'un catalyseur et d'au moins un flux gazeux comprenant de l'hydrogène, caractérisé en ce que tout lit catalytique de la partie interne de ladite zone réactionnelle est traversé à co-courant ascendant par ledit flux gazeux et le liquide. L'invention concerne aussi des procédés d'hydrogénation sélective des hydrocarbures insaturés légers, principalement des oléfines éventuelles et du benzène, compris dans un mélange constitué en majeure partie d'hydrocarbures comportant au moins cinq atomes de carbone par molécule, ainsi que d'hydroisomérisation d'au moins une partie du butène-1 compris dans une charge comprenant en majeure partie des hydrocarbures oléfiniques dont de l'isobutène, ainsi que du butène-1 et des butènes-2 en rapport correspondant sensiblement à l'équilibre thermodynamique.

Outre l'hydrogénation sélective des composés insaturés légers d'un réformat, dont les oléfines et le benzène, sans hydrogénation notable de composés insaturés plus lourds tels que le toluène et, a fortiori, les xylènes, le dispositif selon l'invention peut s'appliquer à diverses réactions catalytiques, équilibrées ou complètes, pour lesquelles on peut séparer, par distillation, au moins l'un des produits de la réaction, à l'état pur ou dilué, dans les conditions de température et de pression voisines de celles de la réaction, et plus particulièrement aux réactions d'isomérisation de paraffines par réorganisation du squelette, d'isomérisation d'oléfines par déplacement de la double liaison (hydroisomérisation) ou par réorganisation du squelette, d'hydrogénation de composés insaturés en composés saturés, de déshydrogénation de composés saturés en composés insaturés, toutes réactions qui nécessitent la présence d'hydrogène.

Le catalyseur d'hydrogénation peut être disposé dans la zone réactionnelle suivant les différentes technologies proposées pour conduire des distillations catalytiques. Ces technologies ont été développées principalement pour les réactions d'éthérification, qui impliquent le contact entre des réactifs en phase liquide homogène et le catalyseur solide. Elles sont essentiellement de deux types. Suivant le premier type de technologies, la réaction et la distillation procèdent simultanément dans le même espace physique, comme l'enseignent par exemple la demande de brevet WO-A-90/02.603, les brevets US-A-4.471.154, US-A-4.475.005, US-A-4.215.011, US-A-4.307.254, US-A-4.336.407, US-A-4.439.350, US-A-5.189.001, US-A-5.266.546, US-A-5.073.236, US-A-5.215.011, US-A-5.275.790, US-A-5.338.517, US-A-5.308.592, US-A-5.236.663, US-A-5.338.518, ainsi que les brevets EP-B1-0.008.860, EP-B1-0.448.884, EP-B1-0.396.650 et EP-B1-0.494.550 et la demande de brevet EP-A1-0.559.511. Le catalyseur est alors généralement en contact avec une phase liquide descendante, générée par le reflux introduit au sommet de la zone de distillation, et avec une phase vapeur ascendante, générée par la vapeur de rebouillage introduite en fond de zone. Suivant le second type de technologies, le catalyseur est disposé de telle façon que la réaction et la distillation procèdent généralement de manière indépendante et consécutive, comme l'enseignent par exemple les brevets US-A-4.847.430, US-A-5.130.102 et US-A-5.368.691, la vapeur de la distillation ne traversant pratiquement pas tout lit catalytique de la zone réactionnelle.

Pour toute réaction chimique nécessitant l'apport d'un réactif gazeux étranger à la charge de la distillation, ce réactif devra être introduit dans la zone réactionnelle de manière différente suivant le type de technologies choisi pour conduire la distillation catalytique. Suivant le premier type de technologies, le réactif gazeux peut être simplement adjoint à la vapeur de la distillation à un niveau quelconque, mais en tout état de cause, avant sa pénétration dans la zone réactionnelle, généralement sensiblement à l'entrée d'au moins un lit catalytique de la zone réactionnelle. Suivant le second type de technologies, le réactif gazeux doit être introduit d'une manière appropriée aux options choisies pour imposer le sens de circulation du liquide et du gaz dans le lit de catalyseur.

Il est apparu donc que, pour une réaction intervenant en présence d'un catalyseur solide, entre un réactif en phase liquide et un réactif gazeux peu soluble dans le liquide, telle que l'hydrogénation d'hydrocarbures insaturés en mélange avec d'autres hydrocarbures, l'option qui consiste à conduire la distillation catalytique en évitant le passage de la vapeur de distillation sur le catalyseur et en faisant circuler le liquide et le réactif gazeux à co-courant ascendant dans le lit catalytique est la plus efficace. Il est apparu que la perte de charge au travers du ou des lit(s) catalytique(s) selon le premier type de technologie ne permet pas l'obtention d'un mélange intime entre la phase liquide et le flux gazeux contenant de l'hydrogène. En effet selon ce type de technologie où la réaction et la distillation procédent simultanément dans le même espace physique, la phase liquide descend au travers du lit catalytique en écoulement ruisselant, donc en filets de liquide. La fraction gazeuse contenant la fraction de charge vaporisée et le flux gazeux contenant de l'hydrogène montent au travers du lit catalytique dans des colonnes de gaz. Par cette disposition, l'entropie du système est la plus forte et la perte de charge à travers le (ou les) lit(s) catalytique(s) est la plus faible. Ainsi, la façon d'opérer selon le premier type de technologie ne permet pas facilement de promouvoir la dissolution de l'hydrogène dans la phase liquide comprenant le (ou les) composé(s) insaturé(s).

Le second type de technologie, comportant un dispositif spécifique pour la distribution de la fraction liquide à hydrogéner et le flux gazeux contenant de l'hydrogène, où ladite fraction liquide et ledit flux gazeux traversent le lit catalytique à co-courant ascendant, permet d'effectuer la réaction d'hydrogénation désirée pratiquement en l'absence de la fraction gazeuse de la charge et dans des conditions où la perte de charge à travers le (ou les) lits(s) catalytique(s) est la plus élevée. L'augmentation de la perte de charge, grâce au dispositif spécifique selon l'invention, permet d'augmenter la solubilité de l'hydrogène dans la phase liquide et donc de promouvoir l'hydrogénation dans la fraction liquide.

D'autre part, compte tenu de la nocivité reconnue du benzène et des oléfines, composés insaturés, la tendance générale est de réduire la teneur de ces constituants dans les essences. En effet, le benzène a des propriétés cancérigènes et il est par conséquent exigé de limiter au maximum toute possibilité de polluer l'air ambiant, notamment en l'excluant pratiquement des carburants automobiles. Aux Etats-Unis les carburants reformulés ne doivent pas contenir plus de 1% de benzène; en Europe, même si les spécifications ne sont pas encore aussi sévères, il est préconisé de tendre progressivement vers cette valeur. De plus, les oléfines ont été reconnues comme étant parmi les hydrocarbures les plus réactifs dans le cycle de réactions photochimiques avec les oxydes d'azote, qui se produit dans l'atmosphère et qui conduit à la formation d'ozone. Une élévation de la concentration d'ozone dans l'air peut être source de troubles respiratoires. La diminution de la teneur en oléfines des essences, et plus particulièrement des oléfines les plus légères qui ont le plus tendance à se volatiliser lors des manipulations du carburant, est par conséquent souhaitable.

La teneur en benzène d'une essence est très largement dépendante de celle de la composante réformat de cette essence. Le réformat résulte d'un traitement catalytique de naphta destiné à produire des hydrocarbures aromatiques, comprenant principalement de 6 à 9 atomes de carbone dans leur molécule et dont l'indice d'octane très élevé confère à l'essence ses propriétés antidétonantes. Pour les raisons de nocivité décrites ci-dessus, il est donc nécessaire de réduire au maximum la teneur en benzène du réformat. Plusieurs voies sont envisageables.

Une première voie consiste à limiter, dans le naphta constituant la charge d'une unité de reformage catalytique, la teneur en précurseurs du benzène, tels que le cyclohexane et le méthylcyclopentane. Cette solution permet effectivement de réduire sensiblement la teneur en benzène de l'effluent de l'unité de réformage mais ne peut suffire à elle seule lorsqu'il s'agit de descendre à des teneurs aussi basses que 1 %. Une seconde voie consiste à éliminer, par distillation, une fraction légère du réformat contenant le benzène. Cette solution conduit à une perte de l'ordre de 15 à 20% d'hydrocarbures qui seraient valorisables dans les essences. Une troisième voie consiste à extraire le benzène présent dans l'effluent de l'unité de réformage. Plusieurs techniques connues sont en principe applicables : extraction par solvant, distillation extractive, adsorption. Aucune de ces techniques n'est appliquée industriellement, car aucune ne permet d'extraire sélectivement le benzène d'une manière économique. Une quatrième voie consiste à transformer chimiquement le benzène pour le convertir en un constituant non visé par les limitations légales. L'alkylation par l'éthylène par exemple transforme le benzène principalement en éthylbenzène. Cette opération est cependant onéreuse du fait de l'intervention de réactions secondaires qui nécessitent des séparations coûteuses en énergie.

Le benzène d'un réformat peut également être hydrogéné en cyclohexane. Comme il est impossible d'hydrogéner sélectivement le benzène d'un mélange d'hydrocarbures contenant également du toluène et des xylènes, il est donc nécessaire de fractionner préalablement ce mélange de manière à isoler une coupe ne contenant que le benzène, qui peut alors être hydrogéné. Il a également été décrit un procédé dans lequel le catalyseur d'hydrogénation du benzène est inclus dans la zone de rectification de la colonne de distillation qui sépare le benzène des autres aromatiques (Benzène Réduction - Kerry Rock and Gary Gildert CDTECH - 1994 Conference on Clean Air Act Implementation and Reformulated Gasoline - Oct. 94 ), ce qui permet de réaliser une économie d'appareillage.

De plus, l'isobutène destiné à la polymérisation doit avoir un niveau de pureté supérieur à 99 % et ne plus contenir que des traces de butène-1 et de butènes-2 (quelques dizaines de partie par million en poids, ppm). En effet, si le taux d'impureté dans l'isobutène est trop élevé, les polymères obtenus sont de moins bonne qualité et le rendement de la polymérisation est plus faible. Il est donc nécessaire d'éliminer d'une coupe d'hydrocarbures contenant de l'isobutène les autres hydrocarbures oléfiniques comportant 4 atomes de carbone par molécule. Le butène-1 et l'isobutène ayant des points d'ébullition très voisins, il n'est pas possible de les séparer par distillation à moins de mettre en oeuvre des moyens considérables. Les autres hydrocarbures oléfiniques comportant 4 atomes de carbone peuvent être séparés de l'isobutène par distillation. Le principal problème qui se pose pour produire de l'isobutène haute pureté est donc la séparation du butène-1 de l'isobutène. Pour effectuer cette séparation plusieurs voies sont envisageables.

La première voie consiste en une extraction à l'acide sulfurique : l'isobutène est hydraté sélectivement et régénéré ensuite par traitement de la phase aqueuse. Si la température et la concentration sont bien contrôlés, ce procédé permet d'obtenir de l'isobutène de bonne pureté. Cependant, le rendement n'excède habituellement pas 90 %, l'extraction n'étant pas complète et il se forme des dimères et oligomères, conduisant à la formation de boues acides toxiques. La deuxième voie consiste en un craquage de l'éther méthylique de l'alcool tertio-butylique (MTBE) : l'isobutène est extrait de la coupe C₄ en le faisant réagir avec du méthanol afin de former du MTBE. Le MTBE est alors craqué en méthanol et isobutène sur un catalyseur acide. Le rendement de récupération peut-être d'au moins 96 %. L'isobutène produit est de bonne pureté mais il doit être débarrassé du diméthyléther qui peut se former durant le craquage. La troisième voie envisageable est la déshydratation de l'alcool butylique tertiaire (ABT). Dans l'opération précédente, le méthanol peut être remplacé par l'eau, ce qui conduit à la production d'ABT. L'isobutène est ensuite récupéré par déshydratation de l'ABT. Cette voie n'est pratiquement pas utilisée, essentiellement parce que l'ABT est très lié au marché de l'oxyde de propylène. L'ABT peut, selon les procédés, être un sous-produit de l'oxyde de propylène.

Le brevet US-A-2.403.672 décrit un procédé de séparation d'isobutène à partir d'un mélange isobutène-butène-1 qui comprend l'introduction du mélange dans une zone d'isomérisation et de fractionnement dans laquelle le catalyseur d'isomérisation joue également le rôle de corps de garnissage assurant la fonction de distillation. Cette solution présente l'inconvénient majeur de ne pas avoir une bonne efficacité de distillation et donc une médiocre capacité de séparation de l'isobutène du butène-1. Selon cette technologie la réaction et la distillation procèdent simultanément dans le même espace physique. Le catalyseur est en contact avec une phase liquide descendante, générée par le reflux introduit au sommet de la zone de distillation, et avec une phase vapeur ascendante, générée par la vapeur de rebouillage introduite en fond de zone.

L'invention concerne un dispositif de distillation réactive comportant une zone de distillation, comportant une zone d'épuisement et une zone de rectification, associée à une zone réactionnelle, au moins en partie interne à ladite zone de distillation et comprenant au moins un moyen de confinement d'au moins un lit catalytique permettant de transformer une charge en présence d'un catalyseur, ledit dispositif comprenant au moins un moyen de circulation de la majeure partie du liquide de distillation de bas en haut à travers le catalyseur pour tout lit catalytique de la partie de ladite zone réactionnelle interne à ladite zone de distillation ; et au moins un moyen de circulation de la majeure partie de la vapeur de distillation de bas en haut à travers tout lit catalytique de la partie de ladite zone réactionnelle interne à ladite zone de distillation de telle manière que ladite vapeur de distillation ne soit pratiquement pas en contact avec le catalyseur ; ledit dispositif étant caractérisé en ce que, pour permettre d'effectuer une transformation de ladite charge par réaction catalytique en présence d'au moins un flux gazeux comprenant de l'hydrogène, il comprend en outre au moins un moyen d'introduction dudit flux gazeux dans ladite zone réactionnelle et au moins un moyen de distribution de la majeure partie dudit flux gazeux du bas vers le haut à travers le catalyseur de tout lit catalytique de la partie de ladite zone réactionnelle interne à ladite zone de distillation.

La charge qui alimente la zone de distillation est introduite dans ladite zone généralement au moins à un niveau de ladite zone, de préférence principalement à un seul niveau de ladite zone.

La zone de distillation comprend généralement au moins une colonne munie d'au moins un interne de distillation choisi dans le groupe formé par les plateaux, les garnissages en vrac et les garnissages structurés, ainsi qu'il est connu de l'homme du métier, et tel que l'efficacité globale totale est généralement au moins égale à cinq étages théoriques. Dans les cas connus de l'homme du métier où la mise en oeuvre d'une seule colonne pose des problèmes, on préfère généralement scinder ladite zone de façon à utiliser finalement au moins deux colonnes qui, mises bout à bout, réalisent ladite zone, c'est-à-dire que les zones de rectification, éventuellement réactionnelle et d'épuisement se répartissent sur les colonnes. En pratique, lorsque la zone réactionnelle est au moins en partie interne à la zone de distillation, la zone de rectification ou la zone d'épuisement, et de préférence la zone d'épuisement, peut généralement se trouver dans au moins une colonne différente de la colonne comprenant la partie interne de la zone réactionnelle.

Le moyen de circulation de la vapeur de distribution du bas vers le haut à travers le lit catalytique traverse le niveau de la zone réactionnelle où est situé le lit catalytique, c'est-à-dire qu'il est généralement situé au sein du lit catalytique, mais qu'il peut être aussi situé à la périphérie dudit lit catalytique.

La zone réactionnelle comprend généralement au moins un lit catalytique, de préférence de 2 à 6, de façon encore plus préférée de 2 à 4 lit(s) catalytique(s) ; dans le cas où au moins deux lits catalytiques se trouvent incorporés dans la zone réactionnelle, ces deux lits sont éventuellement séparés par au moins un interne de distillation.

Le dispositif selon l'invention est généralement tel que l'écoulement du liquide à transformer est co-courant à l'écoulement du flux gazeux comprenant de l'hydrogène et tel que la vapeur de distillation ne traverse pratiquement pas tout lit catalytique de la partie interne de la zone réactionnelle (ce qui se traduit en pratique par le fait que ladite vapeur est séparée dudit liquide), pour tout lit catalytique de la partie interne de la zone réactionnelle. Dans tous les cas de ce second type de technologies, tout lit catalytique de la partie de la zone réactionnelle qui est dans la zone de distillation est généralement tel que le flux gazeux comprenant de l'hydrogène et le flux du liquide qui va réagir circulent à co-courant, généralement ascendant, à travers ledit lit, même si globalement, dans la zone de distillation catalytique, le flux gazeux comprenant de l'hydrogène et le flux du liquide qui va réagir circulent à contre-courant. De tels systèmes comportent généralement au moins un dispositif de distribution de liquide qui peut être par exemple un répartiteur de liquide, dans tout lit catalytique de la partie interne de la zone réactionnelle. Néanmoins, dans la mesure où les technologies utilisées dans le procédé selon l'invention ont été conçues pour des réactions catalytiques intervenant entre des réactifs liquides, elles ne peuvent convenir sans modification pour une réaction catalytique, pour laquelle l'un des réactifs, l'hydrogène, est à l'état gazeux. Pour tout lit catalytique de la partie interne de la zone réactionnelle, il est donc généralement nécessaire d'adjoindre un dispositif d'introduction du flux gazeux comprenant de l'hydrogène, par exemple selon les techniques décrites ci-après.

Ainsi, pour tout lit catalytique de la partie interne de la zone réactionnelle, la partie interne de la zone réactionnelle comporte au moins un dispositif de distribution de liquide, généralement situé au-dessous dudit lit catalytique, et au moins un dispositif d'introduction du flux gazeux, généralement situé au-dessous ou au sein dudit lit catalytique, de préférence dans ce dernier cas non loin du dispositif d'introduction du liquide. Selon une technique, le dispositif d'introduction du flux gazeux dans tout lit catalytique est identique au dispositif de distribution de liquide dans le lit catalytique, c'est-à-dire qu'il existe un moyen d'introduction du gaz dans le liquide en amont du dispositif de distribution de liquide (par rapport au sens de circulation du liquide). En pratique et en langage courant, ceci revient à réaliser un piquage du gaz dans le liquide en amont du moyen de distribution de liquide. Selon une autre technique, le dispositif d'introduction du flux gazeux est disposé sensiblement au niveau du dispositif de distribution de liquide, le gaz et le liquide étant introduits de façon séparée dans le lit catalytique. Selon cette autre technique, le dispositif d'introduction du flux gazeux est disposé au dessous ou au sein du lit catalytique, de préférence non loin du dispositif de distribution de liquide.

D'autre part, le dispositif selon l'invention est, selon un mode de réalisation de l'invention, tel que ledit flux gazeux comprend en majeure partie de l'hydrogène, l'hydrogène provenant en majeure partie, de préférence en quasi totalité, de l'extérieur de la zone de distillation.

Le dispositif selon l'invention est généralement tel que, pour la partie de la zone réactionnelle interne à la zone de distillation, la charge de la zone réactionnelle est prélevée à la hauteur d'un niveau de prélèvement et représente au moins une partie, de préférence la majeure partie, du liquide coulant dans la zone de distillation, de préférence coulant dans la zone de rectification et de façon encore plus préférée coulant à un niveau intermédiaire de la zone de rectification, l'effluent de la zone réactionnelle étant au moins en partie, de préférence en majeure partie, réintroduit dans la zone de distillation sensiblement à proximité, c'est-à-dire généralement sensiblement à la même hauteur ou sensiblement au-dessus ou sensiblement au-dessous, le plus souvent sensiblement à la même hauteur ou sensiblement au-dessus, c'est-à-dire situé à une distance correspondant à une hauteur comprise entre 0 et 4 plateaux théoriques d'un niveau de prélèvement, de préférence dudit niveau de prélèvement, ceci de manière à assurer la continuité de la distillation. Alors, pour la partie de la zone réactionnelle interne à la zone de distillation, le prélèvement de liquide est fait naturellement par écoulement dans la partie de la zone réactionnelle interne à la zone de distillation, et la réintroduction de liquide en zone de distillation se fait aussi naturellement par écoulement du liquide à partir de la partie de la zone réactionnelle interne à la zone de distillation.

Généralement, le dispositif selon l'invention comprend de 1 à 4 niveau(x) de prélèvement qui alimente(nt) la partie externe de la zone réactionnelle, lorsque la zone réactionnelle n'est pas en totalité interne à la zone de distillation. Généralement, le liquide qui va réagir, soit partiellement, soit totalement, circule d'abord dans la partie externe de la zone réactionnelle puis la partie interne de ladite zone. Alors, deux cas peuvent se présenter. Dans le premier cas, la partie externe de la zone réactionnelle est alimentée par un seul niveau de prélèvement, et alors, si ladite partie comprend plus de deux réacteurs, ceux-ci sont disposés en série ou en parallèle. Dans le second cas, préféré selon la présente invention, la partie externe de la zone réactionnelle est alimentée par au moins deux niveaux de prélèvement.

Le dispositif selon l'invention est, selon un des modes de réalisation préférés de l'invention, tel que la zone réactionnelle est en totalité interne à la zone de distillation.

Le dispositif selon l'invention est, selon un des modes de réalisation préférés de l'invention, tel que la partie interne de la zone réactionnelle est au moins en partie incorporée dans la zone de rectification de la zone de distillation.

Une des réalisations préférées du dispositif selon l'invention est telle que le catalyseur est disposé dans la zone réactionnelle suivant le dispositif de base décrit dans le brevet US-A-5.368.691, aménagé de manière que tout lit catalytique soit alimenté par le flux gazeux comprenant de l'hydrogène, régulièrement distribué à sa base, par exemple selon l'une des techniques décrites ci-avant. Suivant cette technologie, si la zone de distillation comprend une seule colonne et si la zone réactionnelle est en totalité interne à ladite colonne, le catalyseur compris dans tout lit catalytique, interne à la zone de distillation, est alors en contact avec une phase liquide ascendante, générée par le reflux introduit au sommet de la zone de distillation, et avec l'hydrogène qui circule dans le même sens que le liquide; le contact avec la phase vapeur de la distillation est évité en faisant transiter cette dernière par au moins une cheminée spécialement aménagée.

L'invention concerne aussi un procédé de traitement d'une charge, constituée en majeure partie par des hydrocarbures comportant au moins 5, de préférence entre 5 et 9, atomes de carbone par molécule, et comprenant au moins un composé insaturé comprenant au plus six atomes de carbone par molécule dont du benzène, tel que l'on traite ladite charge dans une zone de distillation, comportant une zone d'épuisement et une zone de rectification, associée à une zone réactionnelle d'hydrogénation, au moins en partie interne à ladite zone de distillation, dans laquelle on réalise l'hydrogénation d'au moins une partie, de préférence la majeure partie, des composés insaturés comprenant au plus six atomes de carbone par molécule, c'est-à-dire comprenant jusqu'à six (inclus) atomes de carbone par molécule, et contenus dans la charge, en présence d'un catalyseur d'hydrogénation et d'au moins un flux gazeux comprenant, de préférence en majeure partie, de l'hydrogène, de façon à sortir finalement en tête de la zone de distillation un effluent appauvri en composés insaturés comprenant au plus six atomes de carbone par molécule, et en fond de zone de distillation un effluent appauvri en composés insaturés comprenant au plus six atomes de carbone par molécule, caractérisé en ce que tout lit catalytique de la partie interne de la zone d'hydrogénation est traversé à co-courant ascendant par ledit flux gazeux et le liquide et n'est pratiquement pas traversé par la vapeur de distillation.

La zone réactionnelle d'hydrogénation réalise au moins partiellement l'hydrogénation du benzène présent dans la charge, généralement de telle façon que la teneur en benzène de l'effluent de tête soit au maximum égale à une certaine teneur, et ladite zone réactionnelle réalise au moins en partie, de préférence en majeure partie, l'hydrogénation de tout composé insaturé comprenant au plus six atomes de carbone par molécule et différent du benzène, éventuellement présent dans la charge.

De préférence, le procédé selon l'invention comporte l'utilisation du dispositif selon l'invention.

La zone de distillation, ainsi que les caractéristiques du flux gazeux, de la zone réactionnelle etc ont été décrites précédemment dans le cas du dispositif selon l'invention.

Le procédé selon l'invention est, selon un mode de réalisation de l'invention, tel que l'effluent de fond de la zone de distillation est mélangé à l'effluent de tête de ladite zone. Dans un tel cas, le mélange ainsi obtenu est, après stabilisation éventuelle, utilisé comme carburant soit directement, soit par incorporation aux fractions carburants.

Pour la réalisation de l'hydrogénation selon le procédé selon l'invention, le rapport molaire théorique d'hydrogène nécessaire pour la conversion désirée du benzène est de 3. La quantité d'hydrogène injecté avant ou dans la zone d'hydrogénation est éventuellement en excès par rapport à cette stoechiométrie, et ce d'autant plus que l'on doit hydrogéner en plus du benzène présent dans la charge au moins partiellement tout composé insaturé comprenant au plus six atomes de carbone par molécule et présent dans ladite charge. Si les conditions sont telles qu'il y ait excès d'hydrogène, l'hydrogène en excès peut être avantageusement récupéré par exemple selon l'une des techniques décrites ci-après. Par exemple l'hydrogène en excès qui sort en tête de zone de distillation est récupéré, puis injecté en amont des étapes de compression associées à une unité de réformage catalytique, en mélange avec de l'hydrogène provenant de ladite unité, ladite unité opérant de préférence à basse pression (soit généralement une pression inférieure à 8 bar). Cet hydrogène en excès peut aussi être récupéré, puis comprimé et réutilisé dans ladite zone réactionnelle.

L'hydrogène utilisé dans la zone réactionnelle selon l'invention provient généralement en majeure partie, de préférence en quasi totalité, de l'extérieur de la zone de distillation. Il peut provenir de toute source produisant de l'hydrogène à au moins 50 % volume de pureté, de préférence au moins 80 % volume de pureté et de préférence encore au moins 90 % volume de pureté. Par exemple, on peut citer l'hydrogène provenant des procédés de réformage catalytique, de P.S.A. (adsorption par alternance de pression), de génération électrochimique, de vapocraquage ou de réformage à la vapeur.

Les conditions opératoires de la zone d'hydrogénation dans le cas du procédé selon l'invention sont liées aux conditions opératoires de la distillation. La distillation est réalisée sous une pression généralement comprise entre 2 et 20 bar, de préférence entre 4 et 15 bar, de façon ecnore plus préférée entre 4 et 10 bar (1 bar = 10⁵ Pa), avec un taux de reflux compris entre 1 et 10, et de préférence compris entre 3 et 6. La température de tête de zone est comprise généralement entre 40 et 180°C et la température de fond de zone est comprise généralement entre 120 et 280°C. La réaction d'hydrogénation est conduite dans des conditions qui sont le plus généralement intermédiaires entre celles établies en tête et en fond de zone de distillation, à une température comprise entre 100 et 200°C, et de préférence comprise entre 120 et 180°C, et à une pression comprise entre 2 et 20 bar, de préférence entre 4 et 10 bar. Le liquide soumis à l'hydrogénation est alimenté par de l'hydrogène dont le débit dépend de la concentration en benzène dans ledit liquide et, plus généralement, des composés insaturés comportant au plus six atomes de carbone par molécule de la charge de la zone de distillation. Il est généralement au moins égal au débit correspondant à la stoechiométrie des réactions d'hydrogénation en jeu (hydrogénation du benzène et des autres composés insaturés comportant au plus six atomes de carbone par molécule, compris dans la charge d'hydrogénation) et au plus égal au débit correspondant à 10 fois la stoechiométrie, de préférence inférieur à 6 fois la stoechiométrie, de manière encore plus préférée, inférieur à 3 fois la stoechiométrie.

Lorsque la zone d'hydrogénation comprend une partie externe à la zone de distillation, le catalyseur disposé dans ladite partie externe l'est suivant toute technologie connue de l'homme de métier dans des conditions opératoires (température, pression...) indépendantes ou non, de préférence indépendantes, des conditions opératoires de la zone de distillation. Dans la partie de la zone d'hydrogénation externe à la zone de distillation, les conditions opératoires sont généralement les suivantes. La pression requise pour cette étape d'hydrogénation est généralement comprise entre 1 et 60 bar absolus, de préférence entre 2 et 50 bar et de façon encore plus préférée entre 5 et 35 bar. La température opératoire de la zone d'hydrogénation est généralement comprise entre 100 et 400 °C, de préférence entre 120 et 350 °C et de façon préférée entre 140 et 320 °C. La vitesse spatiale au sein de ladite zone d'hydrogénation, calculée par rapport au catalyseur, est généralement comprise entre 1 et 50 et plus particulièrement entre 1 et 30 h⁻¹ (volume de charge par volume de catalyseur et par heure). Le débit d'hydrogène correspondant à la stoechiométrie des réactions d'hydrogénation en jeu est compris entre 0,5 et 10 fois ladite stoechiométrie, de préférence entre 1 et 6 fois ladite stoechiométrie et de façon encore plus préférée entre 1 et 3 fois ladite stoechiométrie. Mais les conditions de température et de pression peuvent aussi, dans le cadre du procédé de la présente invention, être comprises entre celles qui sont établies en tête et en fond de zone de distillation.

De façon plus générale, quelle que soit la position de la zone d'hydrogénation par rapport à la zone de distillation, le catalyseur utilisé dans la zone d'hydrogénation selon l'invention comprend généralement au moins un métal choisi dans le groupe formé par le nickel et le platine, utilisé tel quel ou de préférence déposé sur un support. Le métal doit généralement se trouver sous forme réduite au moins pour 50 % en poids de sa totalité. Mais tout autre catalyseur d'hydrogénation connu de l'homme du métier peut également être choisi.

Lors de l'utilisation du platine, le catalyseur peut contenir avantageusement au moins un halogène dans une proportion en poids par rapport au catalyseur comprise entre 0,2 et 2 %. De manière préférée, on utilise le chlore ou le fluor ou la combinaison des deux dans une proportion par rapport au poids total de catalyseur comprise entre 0,2 et 1,5 %. Dans le cas de l'utilisation d'un catalyseur contenant du platine, on utilise généralement un catalyseur tel que la taille moyenne des cristallites de platine est inférieure à 60.10⁻¹⁰ m, de préférence inférieure à 20.10⁻¹⁰ m, de façon encore plus préférée inférieure à 10.10⁻¹⁰ m. De plus, la proportion totale de platine par rapport au poids total de catalyseur est généralement comprise entre 0,1 et 1 % et de façon préférée entre 0,1 et 0,6 %.

Dans le cas de l'utilisation du nickel, la proportion de nickel par rapport au poids total de catalyseur est comprise entre 5 et 70 %, plus particulièrement entre 10 et 70 % et de façon préférée entre 15 et 65 %. De plus, on utilise généralement un catalyseur tel que la taille moyenne des cristallites de nickel est inférieure à 100.10⁻¹⁰ m, de préférence inférieure à 80.10⁻¹⁰ m, de façon encore plus préférée inférieure à 60.10 ¹⁰ m.

Le support est généralement choisi dans le groupe formé par l'alumine, les silice-alumines, la silice, les zéolithes, le charbon actif, les argiles, les ciments alumineux, les oxydes de terres rares et les oxydes alcalino-terreux, seuls ou en mélange. On utilise de préférence un support à base d'alumine ou de silice, de surface spécifique comprise entre 30 et 300 m²/g, de préférence entre 90 et 260 m²/g.

L'invention concerne enfin un procédé de traitement d'une charge, comprenant en majeure partie des hydrocarbures oléfiniques comportant 4 atomes de carbone par molécule dont de l'isobutène, ainsi que du butène-1 et des butènes-2 en rapport correspondant sensiblement à l'équilibre thermodynamique, dans lequel on traite ladite charge dans une zone de distillation, comportant une zone d'épuisement et une zone de rectification, associée à une zone réactionnelle d'hydroisomérisation, ladite zone réactionnelle étant au moins en partie interne à ladite zone de distillation et comprenant au moins un lit catalytique, dans laquelle on réalise l'hydroisomérisation d'au moins une partie, de préférence la majeure partie du butène-1, en présence d'un catalyseur d'hydroisomérisation et d'un flux gazeux comprenant, de préférence en majeure partie, de l'hydrogène, de façon à sortir finalement en tête de la zone de distillation un effluent riche en isobutène, généralement de haute pureté, et en fond de zone de distillation un effluent appauvri en isobutène, ledit procédé étant caractérisé en ce que tout lit catalytique de la partie interne de la zone d'hydroisomérisation est traversé à co-courant ascendant par ledit flux gazeux et le liquide et n'est pratiquement pas traversé par la vapeur de distillation. Ledit procédé permet la production d'isobutène de haute pureté.

De préférence, le procédé selon l'invention comporte l'utilisation du dispositif selon l'invention.

La charge qui alimente la zone de distillation est introduite dans ladite zone généralement au moins à un niveau de ladite zone, de préférence principalement à un seul niveau de ladite zone. Elle est en rapport correspondant sensiblement à l'équilibre thermodynamique butène-1 - butènes-2 à l'introduction. Une des mises en oeuvre préférée du procédé selon l'invention comprend l'obtention de ladite charge à partir d'une coupe comprenant en majeure partie des hydrocarbures oléfiniques comportant 4 atomes de carbone par molécule, dont de l'isobutène et du butène-1, par traitement de ladite coupe dans une première zone d'hydroisomérisation, généralement indépendante de la partie externe éventuelle de la zone réactionnelle d'hydroisomérisation associée à la zone de distillation, la majeure partie de l'effluent de ladite première zone d'hydroisomérisation servant alors de charge, principale ou secondaire selon les définitions données ci-après dans le texte, qui alimente la zone de distillation. Si ladite charge comprend des composés polyinsaturés, le plus souvent diéniques et/ou acétyléniques, lesdits composés sont de préférence transformés en butènes par la première zone d'hydroisomérisation avant l'introduction dans la zone de distillation. Mais toute autre technique permettant d'obtenir, à partir d'une coupe comprenant en majeure partie des hydrocarbures oléfiniques en C₄ une charge où le butène-1 et les butènes-2 sont en rapport correspondant sensiblement à l'équilibre thermodynamique est aussi envisageable dans le cadre de l'invention.

La première zone réactionnelle d'hydroisomérisation éventuelle, située en amont de la zone de distillation-réaction, réalise au moins partiellement l'hydrogénation sélective des composés polyinsaturés, le plus souvent diéniques tel que le butadiène, en plus de l'hydroisomérisation d'au moins une partie du butène-1 en butènes-2. Elle comprend généralement au moins un lit catalytique d'hydroisomérisation comprenant un catalyseur d'hydroisomérisation, de préférence de 1 à 4 lit(s) catalytique(s) ; dans le cas où au moins deux lits catalytiques se trouvent incorporés dans ladite zone réactionnelle, ces deux lits sont de préférence répartis dans au moins deux réacteurs, répartis en série ou en parallèle, de préférence en série. Par exemple ladite première zone réactionnelle comprend un seul réacteur dans lequel se trouve au moins un, et de préférence un seul, lit catalytique. Une des mises en oeuvre préférées du procédé de la présente invention est telle que ladite première zone réactionnelle comprend deux réacteurs généralement répartis en série comportant chacun au moins un, de préférence un seul, lit catalytique. Lorsque ladite zone réactionnelle comprend au moins deux réacteurs, le recyclage éventuel d'au moins une partie de l'effluent d'au moins un des réacteurs compris dans la première zone réactionnelle dans ladite première zone est effectué généralement à l'entrée d'un réacteur, de préférence dudit réacteur, de préférence avant l'injection du composé gazeux comprenant de l'hydrogène. Il est aussi possible de procéder à un recyclage autour de ladite première zone elle-même, c'est-à-dire généralement à l'entrée du premier réacteur de ladite zone, de préférence avant l'injection du composé gazeux comprenant de l'hydrogène ; par exemple, dans le cas de deux réacteurs, au moins une partie de l'effluent du second réacteur est recyclée vers l'entrée du premier réacteur. Ceci permet avantageusement d'abaisser la teneur en composés polyinsaturés dans l'effluent de ladite première zone réactionnelle.

Les conditions opératoires de la première zone d'hydroisomérisation, lorsqu'elle est présente, sont généralement les suivantes : le catalyseur est identique au catalyseur de la zone d'hydroisomérisation qui sera décrit ci-après. La pression est généralement comprise entre 4 et 40 bar (1 bar = 0,1 MPa), de préférence entre 6 et 30 bar. La température est généralement comprise entre 10 et 150°C, de préférence entre 20 et 100°C. Le rapport molaire H₂/hydrocarbures est généralement ajusté de façon à obtenir une conversion pratiquement totale des composés polyinsaturés tel que le butadiène et une isomérisation suffisante du butène-1 en butènes-2 avec formation limitée d'alcanes.

La zone réactionnelle d'hydroisomérisation associée à la zone de distillation comprend généralement au moins un lit catalytique d'hydroisomérisation comprenant un catalyseur d'hydroisomérisation, de préférence de 2 à 4, de façon encore plus préférée de 2 à 6 lits catalytiques ; dans le cas où au moins deux lits catalytiques se trouvent incorporés dans ladite zone de distillation, ces deux lits sont de préférence séparés par au moins un interne de distillation. Ladite zone réactionnelle d'hydroisomérisation réalise au moins partiellement l'hydroisomérisation d'au moins une partie, de préférence la maeure partie, du butène-1 présent dans sa charge en butènes-2 (cis et trans), généralement de telle façon que la teneur en butène-1 de l'effluent de tête de la zone de distillation soit au maximum égale à une certaine teneur.

La zone de distillation utilisée dans le procédé selon l'invention est identique à celle décrite précédemment.

Un des mises en oeuvre préférées du procédé selon l'invention comprend l'alimentation de la zone de distillation, en plus de l'alimentation en la charge principale, en une charge dite secondaire (par rapport à la charge principale), qui provient ou non d'une zone réactionnelle d'hydroisomérisation telle que la première zone réactionnelle éventuelle d'hydroisomérisation, indépendamment ou non de l'alimentation de la zone de distillation en la charge principale. La charge secondaire est généralement une coupe C4 contenant au moins de l'isobutène, ainsi que du butène-1 et des butènes-2 en rapport correspondant sensiblement à l'équilibre thermodynamique, et est généralement issue d'un procédé de vapocraquage, telle que la coupe C4 brute ou le raffinat-1, ou de craquage catalytique ; généralement et de façon préférée, la charge secondaire est une coupe C4 essentiellement exempte de composés polyinsaturés et sa teneur en butène-1 est inférieure à la teneur en butène-1 de la charge principale. Si la teneur en composés insaturés de la charge secondaire est élevée, ladite charge est de préférence traitée dans une zone d'hydrogénation sélective avant son entrée dans la zone de distillation.

Lorsque la charge principale est introduite en un seul niveau d'introduction, la charge secondaire est généralement introduite dans la zone de distillation en au moins un niveau d'introduction, de préférence en un seul niveau d'introduction, ledit niveau d'introduction dépendant de la composition de ladite charge secondaire. Ainsi, dans un premier exemple la charge secondaire peut être très riche en isobutène et contenir moins de 1,5 fois de butène-1 que la charge principale n'en contient, auquel cas la charge secondaire est de préférence introduite en un seul niveau situé généralement au-dessus du niveau d'introduction de la charge principale. Ou bien dans un second exemple la charge secondaire peut être pratiquement exempte de butène-1, auquel cas la charge secondaire est de préférence introduite en un seul niveau situé généralement au-dessous du niveau d'introduction de la charge principale. Il est aussi possible de procéder au mélange éventuel de la charge principale, avant son entrée en zone de distillation, et de la charge secondaire.

La zone réactionnelle d'hydroisomérisation associée à la zone de distillation comprend généralement au moins un lit catalytique d'hydroisomérisation, de préférence de 2 à 6, de façon encore plus préférée de 2 à 4 lit(s) catalytique(s) ; dans le cas où au moins deux lits catalytiques se trouvent incorporés dans la zone de distillation, ces deux lits sont éventuellement séparés par au moins un interne de distillation. La zone réactionnelle d'hydroisomérisation réalise au moins partiellement l'hydroisomérisation d'au moins une partie, de préférence de la majeure partie, du butène-1 présent dans la charge en butènes-2 (cis et trans), généralement de telle façon que la teneur en butène-1 de l'effluent de tête soit au maximum égale à une certaine teneur.

Le procédé selon l'invention est généralement tel que l'écoulement du liquide à hydroisomériser est co-courant à l'écoulement du flux gazeux comprenant de l'hydrogène, pour tout lit catalytique de la partie interne de la zone d'hydroisomérisation, et tel que la vapeur de distillation ne traverse pratiquement pas tout lit catalytique de la partie interne de la zone réactionnelle (ce qui se traduit en pratique par le fait que ladite vapeur est séparée dudit liquide à hydroisomériser). Tout lit catalytique de la partie de la zone réactionnelle interne à la zone de distillation est généralement tel que le flux gazeux comprenant de l'hydrogène et le flux du liquide qui va réagir circulent à co-courant, généralement ascendant, à travers ledit lit, même si globalement, dans la zone de distillation catalytique, le flux gazeux comprenant de l'hydrogène et le flux du liquide qui va réagir circulent à contre-courant. De tels systèmes comportent généralement au moins un dispositif de distribution de liquide qui peut être par exemple un répartiteur de liquide, pour tout lit catalytique de la partie interne de la zone réactionnelle. Les dispositifs de distribution du flux gazeux et de répartition du liquide ont été décrits précedemment.

Le procédé selon l'invention est généralement tel que la charge de toute partie de la zone réactionnelle d'hydroisomérisation, qu'elle soit interne ou éventuellement externe, est prélevée à la hauteur d'un niveau de prélèvement et représente au moins une partie, de préférence la majeure partie, du liquide (reflux) coulant dans la zone de distillation, de préférence coulant dans la zone de rectification et de façon encore plus préférée coulant à un niveau intermédiaire de la zone de rectification, l'effluent de la zone réactionnelle d'hydroisomérisation étant au moins en partie, de préférence en majeure partie, réintroduit dans la zone de distillation, de manière à assurer la continuité de la distillation. Pour la partie éventuelle de la zone réactionnelle externe à la zone de distillation, la réintroduction de l'effluent en zone de distillation se fait sensiblement à proximité, c'est-à-dire généralement sensiblement à la même hauteur ou sensiblement au-dessus ou sensiblement au-dessous, le plus souvent sensiblement à la même hauteur ou sensiblement au-dessus, c'est-à-dire situé à une distance correspondant à une hauteur comprise entre 0 et 4 plateaux théoriques d'un niveau de prélèvement, de préférence dudit niveau de prélèvement, ceci pour assurer la continuité de la distillation. Pour la partie de la zone réactionnelle interne à la zone de distillation, le prélèvement de liquide (reflux) est fait naturellement par écoulement dans la partie de la zone réactionnelle interne à la zone de distillation, et la réintroduction de l'effluent en zone de distillation se fait aussi naturellement par écoulement du liquide à partir de la zone réactionnelle interne à la zone de distillation.

Généralement, lorsque la zone d'hydroisomérisation n'est pas en totalité interne à la zone de distillation, le procédé selon l'invention comprend de 1 à 6, de préférence de 2 à 4 niveau(x) de prélèvement qui alimente(nt) la partie externe de la zone d'hydroisomérisation. Dans un tel cas, le liquide à hydroisomériser, soit partiellement, soit totalement, circule d'abord dans la partie externe de la zone d'hydroisomérisation puis dans la partie interne de ladite zone. Alors, deux cas peuvent se présenter. Dans le premier cas, la partie externe de la zone d'hydroisomérisation est alimentée par un seul niveau de prélèvement, et alors, si ladite partie comprend plus de deux réacteurs, ceux-ci sont disposés en série ou en parallèle. Dans le second cas, préféré selon la présente invention, la partie externe de la zone d'hydroisomérisation est alimentée par au moins deux niveaux de prélèvement. Une partie de la partie externe de ladite zone d'hydroisomérisation qui est alimentée par un niveau de prélèvement donné, si ladite partie externe comprend au moins deux niveaux de prélèvement, comprend généralement au moins un réacteur, de préférence un seul réacteur. Si ladite partie de la partie externe comprend au moins deux réacteurs, chaque réacteur externe à la zone de distillation est généralement alimenté par un seul niveau de prélèvement, de préférence associé à un seul niveau de réintroduction, ledit niveau de prélèvement étant distinct du niveau de prélèvement qui alimente l'(les) autre(s) réacteur(s).

Le procédé selon l'invention est, selon un des modes de réalisation préférés de l'invention, tel que la zone d'hydrogénation est en totalité interne à la zone de distillation.

L'hydrogène utilisé selon l'invention pour l'hydroisomérisation du butène-1 provient généralement en majeure partie, de préférence en quasi totalité, de l'extérieur de la zone de distillation. Il peut provenir de toute source produisant de l'hydrogène à au moins 50 % volume de pureté, de préférence au moins 80 % volume de pureté et de préférence encore au moins 90 % volume de pureté. Par exemple, on peut citer l'hydrogène provenant des procédés de réformage catalytique, de P.S.A. (adsorption par alternance de pression), de génération électrochimique, de vapocraquage ou de réformage à la vapeur.

Les conditions opératoires de la partie de la zone d'hydroisomérisation interne à la zone de distillation sont liées aux conditions opératoires de la distillation. La distillation est généralement conduite de manière à minimiser la quantité d'isobutène dans le produit de fond afin de maximiser le rendement du procédé en isobutène et de manière à minimiser la quantité de butènes-2 et butène-1 dans le produit de tête, afin d'avoir en tête de l'isobutène de haute pureté. Elle est réalisée sous une pression généralement comprise entre 2 et 30 bar, de préférence entre 4 et 15 bar, de façon encore plus préférée entre 4 et 10 bar, avec un taux de reflux compris entre 1 et 30, et de préférence compris entre 5 et 20. La température de tête de zone est comprise généralement entre 0 et 200 °C et la température de fond de zone est comprise généralement entre 5 et 250 °C. La réaction d'hydroisomérisation est conduite dans des conditions qui sont le plus généralement intermédiaires entre celles établies en tête et en fond de zone de distillation, à une température comprise entre 20 et 150 °C, et de préférence comprise entre 40 et 80 °C, et à une pression comprise entre 2 et 30 bar, de préférence entre 4 et 15 bar, de façon encore plus préférée entre 4 et 10 bar. Le liquide soumis à l'hydroisomérisation est alimenté par un flux gazeux comprenant, de préférence en majeure partie, de l'hydrogène.

Lorsque la zone d'hydroisomérisation comprend une partie externe à la zone de distillation, le catalyseur disposé dans ladite partie externe l'est suivant toute technologie connue de l'homme de métier dans des conditions opératoires (température, pression...) généralement indépendantes des conditions opératoires de la zone de distillation. Dans la partie éventuelle de la zone d'hydroisomérisation externe à la zone de distillation, les conditions opératoires sont généralement les suivantes. La pression requise pour cette étape d'hydroisomérisation est généralement d'environ 1 à 40 bar absolus, de préférence d'environ 2 à 30 bar et de façon encore plus préférée d'environ 4 à 25 bar. La température opératoire de la zone d'hydroisomérisation est généralement d'environ 20 à 150 °C, de préférence d'environ 40 à 100 °C et de façon préférée d'environ 40 à 80 °C. La vitesse spatiale au sein de ladite zone d'hydroisomérisation, calculée par rapport au catalyseur, est généralement d'environ 1 à 100 et plus particulièrement d'environ 4 à 50 h⁻¹ (volume de charge par volume de catalyseur et par heure). Le débit d'hydrogène correspondant est tel que le rapport molaire H₂/hydrocarbures entrant dans la zone d'hydroisomérisation est de préférence au moins égal à 10⁻⁵. Ce rapport est le plus souvent d'environ 10⁻⁵ à environ 3 et très fréquemment d'environ 10⁻³ à environ 1. Mais les conditions de température et de pression peuvent aussi, dans le cadre du procédé de la présente invention, être comprises entre celles qui sont établies en tête et en fond de zone de distillation.

Pour la réalisation de l'hydroisomérisation selon le procédé de l'invention, le rapport molaire théorique d'hydrogène nécessaire pour la conversion désirée du butène-1 dans la zone réactionnelle associée à la zone de distillation est tel que le rapport molaire H₂/hydrocarbures entrant dans ladite zone est au moins égal à 10⁻⁵. Ce rapport molaire peut-être optimisé de telle manière que d'une part tout l'hydrogène soit consommé dans la réaction d'hydroisomérisation afin d'éviter un dispositif de récupération de l'hydrogène en sortie de ladite zone réactionnelle, d'autre part de minimiser les réactions parasites d'hydrogénation de l'isobutène afin de maximiser le rendement du procédé en isobutène et enfin de telle manière qu'il y a suffisamment d'hydrogène tout le long de ladite zone réactionnelle pour que la réaction d'hydroisomérisation du butène-1 en butènes-2 puisse se faire. Cependant si les conditions sont telles qu'il y ait excès d'hydrogène, l'hydrogène en excès peut être avantageusement récupéré par exemple selon l'une des techniques décrites ci-après. Par exemple l'hydrogène en excès qui sort en tête de zone de distillation est récupéré, puis injecté en amont des étapes de compression associées à une unité de réformage catalytique, en mélange avec de l'hydrogène provenant de ladite unité, ladite unité opérant de préférence à basse pression (soit généralement une pression inférieure à 8 bar). Cet hydrogène en excès peut aussi être récupéré, puis comprimé et réutilisé dans ladite zone réactionnelle.

Dans le cas de la présence d'une partie externe de la zone d'hydroisomérisation associée à la zone de distillation, le procédé selon l'invention permet d'isomériser une grande partie du butène-1 en butènes-2 à l'extérieur de la zone de distillation, éventuellement dans des conditions de pression et/ou de température différentes de celles utilisées dans la colonne. De préférence, la température à l'entrée (respectivement à la sortie) du niveau de prélèvement qui alimente un lit catalytique de la partie de la zone d'hydroisomérisation située à l'extérieur de la colonne, est sensiblement semblable, c'est-à-dire que l'écart est sensiblement inférieur à 10°C par rapport à la température à la hauteur du niveau de prélèvement (respectivement du niveau de réintroduction). De même, on peut avantageusement effectuer la réaction d'hydroisomérisation dans la partie de la zone réactionnelle située à l'extérieur de la colonne à une pression plus élevée que celle utilisée à l'intérieur de la zone de distillation. Cette augmentation de pression permet aussi une dissolution accrue du flux gazeux contenant de l'hydrogène dans la phase liquide contenant le butène-1 à isomériser.

Dans un tel cas, le procédé selon l'invention comprend l'utilisation de la technique dite de "pump-around", c'est-à-dire de pompage en boucle, qui consiste à faire passer à l'extérieur de la zone de distillation une partie, de préférence la majeure partie, du liquide (reflux) par un facteur de préférence supérieur à 1, c'est-à-dire que le débit d'un lit catalytique de la partie externe de la zone d'hydroisomérisation associée à la zone de distillation, ledit lit étant alimenté à un niveau de prélèvement par une partie de l'effluent liquide (reflux) coulant sur le plateau de distillation associé audit niveau de prélèvement (i.e. sur lequel on prélève ladite partie d'effluent liquide) et par au moins une partie du liquide correspondant au recyclage de l'effluent dudit lit sensiblement au-dessus ou sensiblement au-dessous ou sensiblement à la même hauteur que ledit niveau de prélèvement, est supérieur à 1 fois le débit de liquide coulant sur ledit plateau, par exemple égal à 1,5 fois.

De façon plus générale, le catalyseur utilisé dans la zone d'hydroisomérisation selon le procédé de la présente invention comprend généralement au moins un métal choisi dans le groupe formé par les métaux nobles du groupe VIII de la Classification Périodique des éléments et le nickel, c'est-à-dire choisi dans le groupe formé par le ruthénium, le rhodium, le palladium, l'osmium, l'iridium, le platine, de préférence le palladium, ou le nickel, utilisé tel quel ou de préférence déposé sur un support. Le métal doit généralement se trouver sous forme réduite au moins pour 50 % en poids de sa totalité. La teneur en métal noble du catalyseur est habituellement d'environ 0,01 à environ 2 % en poids. Dans le cas de l'utilisation de nickel, la proportion de nickel par rapport au poids total de catalyseur est comprise entre 5 et 70%, et de façon préférée entre 10 et 70 %, et on utilise généralement un catalyseur tel que la taille moyenne des cristallites de nickel est inférieure à 10 nm, de préférence inférieure à 8 nm, de façon encore plus préférée inférieure à 6 nm. Mais tout autre catalyseur d'hydroisomérisation connu de l'homme du métier peut également être choisi. Le catalyseur est habituellement traité par un composé du soufre puis par de l'hydrogène avant son utilisation. Le catalyseur est généralement sulfuré in situ ou ex situ de telle façon que du soufre soit chimisorbé sur une partie au moins du métal. Le soufre chimisorbé a pour effet de favoriser la réaction d'isomérisation du butène-1 en butènes-2 par rapport à la réaction d'hydrogénation de l'isobutène et donc de maximiser le rendement en isobutène du procédé.

Le support du catalyseur d'hydroisomérisation est généralement choisi dans le groupe formé par l'alumine, les silice-alumines, la silice, les zéolithes, le charbon actif, les argiles, les ciments alumineux, les oxydes de terres rares et les oxydes alcalino-terreux, seuls ou en mélange. On utilise de préférence un support à base d'alumine ou de silice, de surface spécifique comprise entre 10 et 300 m²/g, de préférence entre 30 et 70 m²/g.

A titre d'exemple non limitatif utilisable dans le cadre de la présente invention on peut citer les catalyseurs commerciaux tel que celui vendu par la société Catalysts and Chemicals sous la référence C-31, celui vendu par la société Girdler Corporation sous la référence G-55 ou de préférence ceux vendus par la société Procatalyse sous la référence LD-265, LD-265S, LD-267 et LD-267R.

### Exemples

Les exemples 1 et 2 qui suivent montrent la mise en oeuvre d'une zone d'hydrogénation des composés insaturés comprenant au plus six atomes de carbone par molécule dont du benzène, selon l'invention (exemple 2), et la mise en oeuvre d'une zone d'hydrogénation qui n'est pas selon l'invention (exemple 1), avec un catalyseur disposé en vrac sur des plateaux de distillation traversé par le liquide qui circule dans le sens descendant et par la vapeur qui circule dans le sens ascendant de ladite zone de distillation.

### Exemple 1 : (Comparatif)

On utilise une colonne de distillation métallique de diamètre 50 mm, rendue adiabatique par des enveloppes chauffantes dont les températures sont régulées de manière à reproduire le gradient de température qui s'établit dans la colonne. Sur une hauteur de 4,5 m, la colonne comporte, de la tête vers le pied : une zone de rectification composée de 11 plateaux perforés à déversoir et descente, une zone de distillation catalytique hydrogénante et une zone d'épuisement composée de 63 plateaux perforés. La zone de distillation catalytique hydrogénante est constituée de trois plateaux réactifs, qui sont ici des plateaux perforés de distillation, à déversoir et descente, dont les déversoirs sont surélevés de 3,5 cm et dont le volume compris entre le niveau du sommet du déversoir et le plateau peut être garni de catalyseur. Un grillage métallique placé au sommet du déversoir fait office de filtre pour éviter que des particules de catalyseur ne soient évacuées avec le liquide quittant le plateau.

Chacune des trois cellules est garnie de 36 g de catalyseur au nickel vendu par la société PROCATALYSE sous la référence LD 746. Sur le 37ème plateau de la colonne, en partant du fond, on introduit 260 g/h d'un réformat comprenant essentiellement des hydrocarbures ayant au moins 5 atomes de carbone dans leur molécule, dont la composition est présentée dans le tableau 1. On introduit également à la base de chaque cellule un débit de 18 Nl/h d'hydrogène. La colonne est mise en régime en établissant un taux de reflux égal à 5 et en régulant la température de fond à 176°C et la pression à 7 bar.

En régime stabilisé, on recueille à raison de 138 g/h et 113 g/h, respectivement un résidu et un distillat liquide dont les compositions sont données dans le tableau 1. Une faible partie du distillat, constituée des hydrocarbures les plus légers, est évacuée de la colonne avec l'hydrogène excédentaire et n'est pas comptabilisée. Des analyses des effluents on peut déduire que les taux d'hydrogénation des oléfines et du benzène de la charge sont respectivement de 100 et 55%, tandis que le toluène n'est pas affecté.

### Exemple 2 : (selon l'invention)

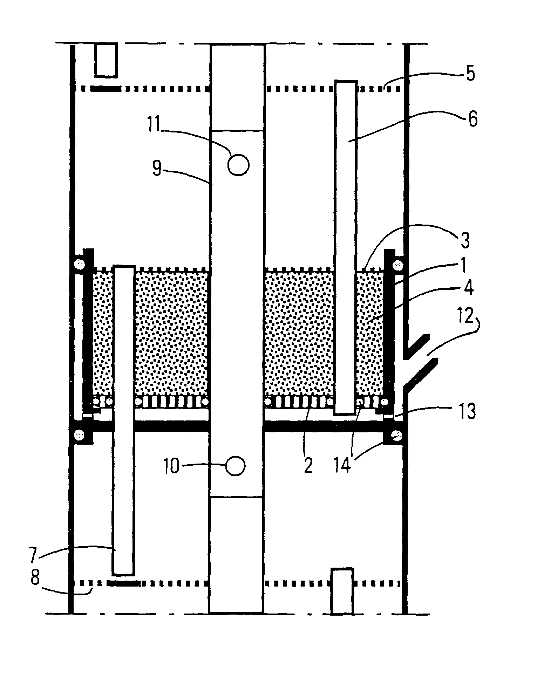

On utilise le même appareillage que celui décrit dans l'exemple 1, mais avec une zone de distillation catalytique de conception différente. La zone de distillation catalytique hydrogénante est ici constituée de trois doublets de distillation catalytique, chaque doublet étant constitué lui-même par une cellule catalytique surmontée de trois plateaux perforés. Le détail de construction d'une cellule catalytique ainsi que sa disposition dans la colonne sont représentés schématiquement sur la figure. La cellule catalytique 1 consiste en un conteneur cylindrique à fond plat, d'un diamètre extérieur inférieur de 2 mm au diamètre intérieur de la colonne. Elle est munie à sa partie inférieure, au dessus du fond, d'une grille 2 qui sert à la fois de support pour le catalyseur et de distributeur pour l'hydrogène, et à sa partie supérieure, d'une grille de retenue du catalyseur 3, dont la hauteur peut être variée. Le catalyseur 4 remplit tout le volume compris entre ces deux grilles. La cellule catalytique reçoit le liquide provenant du plateau de distillation supérieur 5, par la descente 6. Après avoir parcouru la cellule dans le sens ascendant, le liquide est évacué par débordement par la descente 7 et coule sur le plateau de distillation inférieur 8. La vapeur issue du plateau inférieur 8 emprunte la cheminée centrale 9 solidaire de la cellule, en pénétrant par des orifices 10 (un seul apparent sur la figure) et en ressortant sous le plateau supérieur 5 par des orifices 11 (un seul apparent sur la figure). L'hydrogène est introduit au pied de la cellule catalytique par la tubulure 12, puis par les orifices 13 (six au total) répartis sur la périphérie de la cellule, au voisinage immédiat du fond. Des joints d'étanchéité 14 évitent toute fuite d'hydrogène avant son arrivée sur le lit catalytique.

Chacune des trois cellules est garnie de 36 g de catalyseur au nickel vendu par la société PROCATALYSE sous la référence LD 746. Sur le 37ème plateau de la colonne, en partant du fond, on introduit 260 g/h de la même charge que celle utilisée dans l'exemple 1 et dont la composition est présentée dans la deuxième colonne du tableau. On introduit également à la base de chaque cellule un débit de 6 Nl/h d'hydrogène. La colonne est mise en régime en établissant un taux de reflux égal à 5 et en régulant la température de fond à 176°C et la pression à 7 bar.

En régime stabilisé, on recueille à raison de 143 g/h et 106 g/h, respectivement un résidu et un distillat liquide dont les compositions sont données dans le tableau 1. Une faible partie du distillat, constituée des hydrocarbures les plus légers, est évacuée de la colonne avec l'hydrogène excédentaire et n'est pas comptabilisée. Des analyses des effluents on peut déduire que les taux d'hydrogénation des oléfines et du benzène de la charge sont respectivement de 100 et 87%, tandis que le toluène n'est pas affecté.

**Tableau 1 :**

| compositions de la charge et des effluents de la colonne catalytique | | | | | |
|---|---|---|---|---|---|
| | **compositions, en % poids** | | | | |
| | **exemple 1** | | | **exemple 2** | |
| | charge | résidu | distillat liq. | résidu | distillat liq. |
| C5 et plus légers | 7,65 | | 10,22 | | 7,36 |
| dont : oléfines | 0,11 | | 0 | | 0 |
| C6 | 44,83 | 9,55 | 89,78 | 12,4 | 92,59 |
| dont : oléfines | 0,13 | | 0 | | 0 |
| : benzène | 6,07 | 0,63 | 5,45 | 0,07 | 1,84 |
| : cyclohexane | 1,1 | 8,34 | 0,34 | 12,16 | 0,73 |
| C7: | 42,55 | 80,72 | | 78,27 | 0,05 |
| dont : toluène | 4,78 | 9,1 | | 8,87 | |
| C8 et plus lourds | 4,97 | 9,73 | | 9,33 | |
| conversion des oléfines | 100% | | | 100% | |
| conversion du benzène | 55% | | | 87% | |
| conversion de l'hydrogène | 15% | | | 70% | |

II est constaté que le procédé selon la présente invention permet une meilleure conversion du benzène et une meilleure conversion de l'hydrogène.

Les exemples 3 et 4 qui suivent illustrent le cas d'un procédé selon l'invention de traitement d'une charge comprenant en majeure partie des hydrocarbures oléfiniques comportant 4 atomes de carbone par molécule dont de l'isobutène, dont du butène-1 et des butènes-2 en rapport correspondant sensiblement à l'équilibre thermodynamique.

### Exemple 3 :

Les opérations d'hydroisomérisation d'une coupe C₄ et de distillation ont été effectuées successivement de façon discontinue. La charge a été hydroisomérisée une première fois. L'effluent de ce premier essai a été distillé ; la tête de distillation, représentative d'un soutirage intermédiaire, a été hydroisomérisée. L'effluent de l'hydroisomérisation, représentatif de ce qui serait réinjecté dans la colonne, a été distillé. La tête de cette seconde distillation a été hydroisomérisée, et l'effluent de cette troisième hydroisomérisation a été distillé.

Les opérations d'hydroisomérisation sont effectuées dans une unité pilote disposant d'un réacteur adiabatique. Le réacteur est remplie de 1,5 l du catalyseur LD-265 vendu par la société Procatalyse. Le catalyseur est sulfuré et activé in situ selon une procédure préconisée par le fournisseur du catalyseur.

Les opérations de distillation ont été effectuées dans une colonne adiabatique de diamètre interne 163 mm et de hauteur de 10 m. La colonne est constituée de 4 lits de 1,78 m de haut au dessus de l'injection de la charge, remplis d'un garnissage commercialisé par la société Sulzer sous le nom de M550Y et de 2 lits de 1 m de hauteur en dessous de l'injection de la charge, remplis d'anneaux de Pall.

### - Première hydroisomérisation.

Les conditions opératoires moyennes au cours de l'essai sont les suivantes :
- Température du réacteur : 80°C
- Pression du réacteur : 20 bar
- Temps de séjour : 0,25 h.
- Rapport molaire H₂/charge : 3

Le tableau 2 ci-après montre les compositions de la charge et de l'effluent du réacteur d'hydroisomérisation fonctionnant dans les conditions décrites ci-dessus.

**TABLEAU 2**

| | Charge (% poids) | Effluent (% poids) |
|---|---|---|
| < C₄ | 0,25 | 0,23 |
| i C₄ | 2,98 | 3,10 |
| i C₄⁼ | 44,90 | 44,42 |
| C₄⁼ 1 | 26,95 | 4,26 |
| C₄⁼⁼ 1,3 | 0,13 | 0,00 |
| n C₄ | 11,72 | 14,41 |
| C₄⁼ 2 trans | 8,73 | 21,37 |
| Néo C₅ | 0,24 | 0,23 |
| Me Cyclo C₃ | 0,06 | 0,06 |
| C₄⁼2 cis | 4,03 | 11,92 |
| > C₄ | 0,01 | 0,00 |

avec la légende suivante pour ce tableau et pour les tableaux suivants :
- < C₄ :: composés à moins de 4 (4 exclu) atomes de carbone par molécule (ou C3⁻)
- iC4 :: isobutane
- i C₄= :: isobutène
- C₄= 1 :: butène-1
- C₄== 1,3 :: butadiène-1,3
- n C₄ :: normal-butane
- C₄= 2 trans :: butène-2 trans
- Néo C₅ :: néopentane (ou diméthyl propane)
- Me Cyclo C₃ :: méthyl cyclopropane
- C₄= 2 cis :: butène-2 cis
- > C₄ :: composés à plus de 4 (4 exclu) atomes de carbone par molécule (ou C5⁺)

### - Première distillation.

La distillation de l'effluent de l'essai présenté ci dessus a été effectuée avec les conditions opératoires suivantes :
- Pression de la colonne : 4 bar
- Taux de reflux (R/D) : 20
- Température de la charge : 33°C
- Température de reflux : 32°C
- Température en tête de colonne : 57°C
- Température en fond de colonne : 63°C.

Le tableau 3 ci-après montre les compositions de la charge et de l'effluent de tête de la colonne de distillation fonctionnant dans les conditions décrites ci-dessus.

**TABLEAU 3**

| | Charge (% poids) | Tête (% poids) |
|---|---|---|
| < C₄ | 0,23 | 0,44 |
| i C₄ | 3,10 | 6,71 |
| i C₄⁼ | 44,42 | 83,35 |
| C₄⁼ 1 | 4,26 | 7,39 |
| C₄⁼⁼ 1,3 | 0,00 | 0,00 |
| n C₄ | 14,41 | 1,62 |
| C₄⁼ 2 trans | 21,37 | 0,44 |
| Néo C₅ | 0,23 | - |
| Me Cyclo C₃ | 0,06 | - |
| C₄⁼ 2 cis | 11,92 | 0,05 |
| > C₄ | - | - |

### - Deuxième hydroisomérisation.

Les conditions opératoires moyennes au cours de l'essai sont les suivantes :
- Température du réacteur : 65°C
- Pression du réacteur : 20 bar
- Temps de séjour : 0,25 h
- Rapport molaire H₂/charge : 0,6

Le tableau 4 ci-après montre les compositions de la charge et de l'effluent du réacteur d'hydroisomérisation fonctionnant dans les conditions décrites ci-dessus.

**TABLEAU 4**

| | Charge (% poids) | Effluent (% poids) |
|---|---|---|
| < C₄ | 0,44 | 0,39 |
| i C₄ | 6,71 | 6,91 |
| i C₄⁼ | 83,35 | 82,94 |
| C₄⁼ 1 | 7,39 | 0,81 |
| C₄⁼⁼ 1,3 | - | - |
| n C₄ | 1,62 | 2,09 |
| C₄⁼ 2 trans | 0,44 | 4,44 |
| Néo C₅ | - | - |
| Me Cyclo C₃ | - | - |
| C₄⁼ 2 cis | 0,05 | 2,42 |
| > C₄ | - | - |

### - .Deuxième distillation.

La distillation de l'effluent de l'essai présenté ci-dessus a été effectuée avec les conditions opératoires suivantes :
- Pression de la colonne : 4 bar
- Taux de reflux (R/D) : 13,5
- Température de la charge : 36°C
- Température de reflux : 41°C
- Température en tête de colonne : 51°C
- Température en fond de colonne : 55°C.

Le tableau 5 ci-après montre les compositions de la charge et de l'effluent de tête de la colonne de distillation fonctionnant dans les conditions décrites ci-dessus.

**TABLEAU 5**

| | Charge (% poids) | Tête (% poids) |
|---|---|---|
| < C₄ | 0,39 | 0,65 |
| i C₄ | 6,91 | 13,71 |
| i C₄⁼ | 82,94 | 84,82 |
| C₄⁼ 1 | 0,81 | 0,51 |
| C₄⁼⁼ 1,3 | - | - |
| n C₄ | 2,09 | 0,14 |
| C₄⁼ 2 trans | 4,44 | 0,12 |
| Néo C₅ | - | - |
| Me Cyclo C₃ | - | - |
| C₄⁼ 2 cis | 2,42 | 0,05 |
| > C₄ | - | - |

### - Troisième hydroisomérisation.

Les conditions opératoires moyennes au cours de l'essai sont les suivantes :
- Température du réacteur : 60°C
- Pression du réacteur : 20 bar
- Temps de séjour : 0,25 à 0,1 h
- Rapport molaire H₂/charge : 1

Le tableau 6 ci-après montre les compositions de la charge et de l'effluent du réacteur d'hydroisomérisation fonctionnant dans les conditions décrites ci-dessus.

**TABLEAU 6**

| | Charge (% poids) | Effluent (% poids) |
|---|---|---|
| < C₄ | 0,65 | 0,57 |
| i C₄ | 13,71 | 14,55 |
| i C₄⁼ | 84,82 | 84,07 |
| C₄⁼ 1 | 0,51 | 0,03 |
| C₄⁼⁼ 1,3 | - | - |
| n C₄ | 0,14 | 0,22 |
| C₄⁼ 2 trans | 0,12 | 0,38 |
| Néo C₅ | - | - |
| Me Cyclo C₃ | - | - |
| C₄⁼ 2cis | 0,05 | 0,18 |
| > C₄ | - | - |

### - Troisième distillation.

La distillation de l'effluent de l'essai présenté ci-dessus a été effectuée avec les conditions opératoires suivantes :
- Pression de la colonne : 4 bar
- Taux de reflux (R/D) : 13,5
- Température de la charge : 36°C
- Température de reflux : 41°C
- Température en tête de colonne : 53°C
- Température en fond de colonne : 55°C.

Le tableau 7 ci-après montre les compositions de la charge et de l'effluent de tête de la colonne de distillation fonctionnant dans les conditions décrites ci-dessus.

**TABLEAU 7**

| | Charge (% poids) | tête (% poids) |
|---|---|---|
| < C₄ | 0,57 | 0,57 |
| i C₄ | 14,55 | 14,66 |
| i C₄= | 84,07 | 84,69 |
| C₄= 1 | 0,03 | 0,03 |
| C₄== 1,3 | - | - |
| n C₄ | 0,22 | 0,01 |
| C₄= 2 trans | 0,38 | 0,04 |
| Néo C₅ | - | - |
| Me Cyclo C₃ | - | - |
| C₄= 2 cis | 0,18 | - |
| > C₄ | - | - |

Ces opérations successives et discontinues d'hydroisomérisation et de distillation représentent l'opération de séparation du butène-1 de l'isobutène qui est effectuée en continu dans le cas du procédé selon l'invention.

### Exemple 4 :

Des tests pilotes d'hydroisomérisation ont été effectués à partir d'un raffinat-1 sur le catalyseur d'hydroisomérisation LD267R commercialisé par la société Procatalyse qui garnit chacun des lits catalytiques. Les résultats de ces tests sont présentés dans le tableau 8 ci-dessous ; ils ont permis de déterminer les paramètres de calcul qui permettent de simuler le procédé selon l'invention au moyen de logiciels adaptés. Le logiciel utilisé pour cette simulation est commercialisé sous le nom de Pro2 par la société SIMCI.

**TABLEAU 8 :**

| résultats de tests pilotes | | | | | | | | | | | |
|---|---|---|---|---|---|---|---|---|---|---|---|
| T °C | | 40 | 60 | 90 | 50 | 50 | 50 | 50 | 50 | 50 | 50 |
| VVH h-1 | | 30 | 30 | 30 | 30 | 30 | 30 | 30 | 30 | 20 | 40 |
| P bar | | 10 | 10 | 10 | 6,5 | 10 | 15 | 10 | 10 | 10 | 10 |
| H2/H C m/m | | 0,17 | 0,17 | 0,17 | 0,17 | 0,17 | 0,17 | 0,1 | 0,19 | 0,17 | 0,17 |
| | char | effl | effl | effl | effl | effl | effl | effl | effl | effl | effl |
| < C4 | 0,14 | 0,11 | 0,12 | 0,11 | 0,10 | 0,10 | 0,10 | 0,10 | 0,11 | 0,10 | 0,09 |
| iC4 | 5,69 | 5,75 | 5,75 | 5,73 | 5,71 | 5,76 | 5,75 | 5,72 | 5,76 | 5,75 | 5,74 |
| iC4= | 78,6 | 78,7 | 78,7 | 78,7 | 78,7 | 78,7 | 78,7 | 78,7 | 78,7 | 78,7 | 78,7 |
| | 7 | 1 | 2 | 3 | 8 | 2 | 3 | 4 | 2 | 1 | 4 |
| 1-C4= | 3,66 | 1,30 | 0,91 | 0,75 | 1,15 | 1,01 | 1,18 | 1,13 | 1,00 | 0,8 | 1,32 |
| n-C4 | 7,16 | 7,19 | 7,17 | 7,14 | 7,14 | 7,18 | 7,19 | 7,16 | 7,20 | 7,19 | 7,18 |
| tr2-C4= | 4,36 | 5,40 | 5,48 | 5,40 | 5,46 | 5,49 | 5,41 | 5,46 | 5,48 | 5,59 | 5,37 |
| cs2-C4= | 0,32 | 1,54 | 1,85 | 2,14 | 1,66 | 1,74 | 1,64 | 1,69 | 1,75 | 1,86 | 1,56 |
| où char = charge et effl = effluent | | | | | | | | | | | |

La zone de distillation catalytique hydroisomérisante comprend 2 ou 3 doublets de distillation catalytique, chacun de ses doublets étant du type de celui de la figure, chaque doublet étant constitué lui-même par une cellule catalytique surmontée de trois plateaux perforés.

Deux exemples ainsi simulés par le calcul ont été effectués. Ils sont décrits ci-après.

### Exemple 4A :

La configuration de l'unité, comportant trois lits catalytiques d'hydroisomérisation situés à l'intérieur de la colonne, appelés plateaux réactifs, est la suivante :
- colonne de 130 plateaux théoriques, numérotés de haut en bas,
- plateau d'alimentation n° 90,
- les plateaux réactifs sont les plateaux 10, 25 et 39. Ils contiennent chacun 7,5 m³ de catalyseur.

Conditions opératoires :
débit liquide d'alimentation de la colonne : 292,9 kmole/h
taux de reflux : 12,
pression en tête de colonne : 6,2 bar absolu,
pression en fond de colonne : 7 bar absolu,
température de l'alimentation de la colonne : 59 °C,
température en tête de colonne : 52 °C,
température en fond de colonne : 64,5 °C,
température du plateau réactif N° 10 : 53 °C,
pression du plateau réactif N° 10 : 6,6 bar absolu,
débit liquide à travers le plateaux réactif 10 : 1660 kmole/h,
température du plateau réactif N° 25 : 54 °C,
pression du plateau réactif N° 25 6,6 bar absolu,
débit liquide à travers le plateaux réactif 25: 1660 kmole/h,
température du plateau réactif N° 39 : 54 °C,
pression du plateau réactif N° 39 : 6,7 bar absolu,
débit liquide à travers le plateaux réactif 39 : 1660 kmole/h.

Avec cette configuration et sous ces conditions opératoires, la simulation a conduit aux résultats suivants :

| | Alimentation colonne (kmole/h) | Tête de colonne (kmole/h) | Fond de colonne (kmole/h) |
|---|---|---|---|
| < C₄ | 1,12 | 1,12 | 0,00 |
| i C₄ | 4,46 | 5,58 | 0,00 |
| i C₄⁼ | 110,08 | 108,07 | 0,89 |
| C₄⁼ 1 | 7,53 | 0,02 | 0,17 |
| n C₄ | 55,27 | 0,13 | 55,23 |
| C₄⁼ 2 tr | 79,76 | 0,03 | 84,56 |
| C₄⁼ 2 cis | 33,49 | 0,00 | 35,91 |
| H₂ | 1,21 | 0,00 | 0,00 |
| Total | 292,92 | 114,95 | 176,76 |

Rendement en isobutène en tête de colonne : 98,2 %
Rapport molaire butène-1/isobutène en tête de colonne : 1,85 x 10⁻⁴.

### Exemple 4B :

La configuration de l'unité, comportant deux lits catalytiques d'hydroisomérisation situés à l'intérieur de la colonne, appelés plateaux réactifs, est la suivante :
- colonne de 130 plateaux théoriques, numérotés de haut en bas,
- plateau d'alimentation n° 90,
- les plateaux réactifs sont les plateaux 10 et 39. Ils contiennent chacun 7,5 m³ de catalyseurs.

Conditions opératoires :
débit liquide d'alimentation de la colonne : 292,9 kmole/h
taux de reflux : 12,
pression en tête de colonne : 6,2 bar absolu,
pression en fond de colonne : 7 bar absolu,
température de l'alimentation de la colonne : 59 °C,
température en tête de colonne : 52 °C,
température en fond de colonne : 64,5 °C,
température du plateau réactif N° 10 : 53 °C,
pression du plateau réactif N° 10 : 6,6 bar absolu,
débit liquide à travers le plateaux réactif 10 : 1660 kmole/h,
température du plateau réactif N° 39 : 54 °C,
pression du plateau réactif N° 39 : 6,7 bar absolu,
débit liquide à travers le plateaux réactif 39 : 1660 kmole/h.

Avec cette configuration et sous ces conditions opératoires, la simulation a conduit aux résultats suivants :

| | Alimentation colonne (kmole/h) | Tête de colonne (kmole/h) | Fond de colonne (kmole/h) |
|---|---|---|---|
| < C₄ | 1,12 | 1,12 | 0,00 |
| i C₄ | 4,46 | 5,17 | 0,00 |
| i C₄⁼ | 110,08 | 108,48 | 0,89 |
| C₄⁼ 1 | 7,53 | 0,09 | 0,17 |
| n C₄ | 55,27 | 0,13 | 55,20 |
| C₄⁼ 2 tr | 79,76 | 0,06 | 84,50 |
| C₄⁼ 2 cis | 33,49 | 0,00 | 35,90 |
| H₂ | 1,21 | 0,44 | 0,00 |
| Total | 292,92 | 115,49 | 176,66 |

Rendement en isobutène en tête de colonne : 98,6 %
Rapport molaire butène-1/isobutène en tête de colonne : 8,30 x 10⁻⁴.

## Revendications

1. Dispositif de distillation réactive comportant une zone de distillation, comportant une zone d'épuisement et une zone de rectification, associée à une zone réactionnelle, au moins en partie interne à ladite zone de distillation et comprenant au moins un moyen de confinement d'au moins un lit catalytique permettant de transformer une charge en présence d'un catalyseur, ledit dispositif comprenant au moins un moyen de circulation de la majeure partie du liquide de distillation de bas en haut à travers le catalyseur pour tout lit catalytique de la partie de ladite zone réactionnelle interne à ladite zone de distillation ; et au moins un moyen de circulation de la majeure partie de la vapeur de distillation de bas en haut à travers tout lit catalytique de la partie de ladite zone réactionnelle interne à ladite zone de distillation de telle manière que ladite vapeur de distillation ne soit pratiquement pas en contact avec le catalyseur ; ledit dispositif étant **caractérisé en ce que**, pour permettre d'effectuer une transformation de ladite charge par réaction catalytique en présence d'au moins un flux gazeux comprenant de l'hydrogène, il comprend en outre au moins un moyen d'introduction dudit flux gazeux dans ladite zone réactionnelle et au moins un moyen de distribution de la majeure partie dudit flux gazeux du bas vers le haut à travers le catalyseur de tout lit catalytique de la partie de ladite zone réactionnelle interne à ladite zone de distillation.

2. Dispositif selon la revendication 1 tel que, pour chaque lit catalytique de la partie interne de la zone réactionnelle, le moyen de distribution du liquide est situé au-dessous du lit catalytique et le moyen de distribution du flux gazeux est situé au-dessous ou au sein du lit catalytique.

3. Dispositif selon l'une des revendications 1 et 2 tel que le dispositif d'introduction du flux gazeux dans tout lit catalytique est identique au dispositif de distribution de liquide dans le lit catalytique, c'est-à-dire qu'il existe un moyen d'introduction du gaz dans le liquide en amont du dispositif de distribution de liquide par rapport au sens de circulation du liquide.

4. Dispositif selon l'une des revendications 1 et 2 tel que le dispositif d'introduction du flux gazeux est disposé sensiblement au niveau du dispositif de distribution de liquide, le gaz et le liquide étant introduits de façon séparée dans le lit catalytique.

5. Dispositif selon la revendication 4 tel que le dispositif d'introduction du flux gazeux est disposé au sein du lit catalytique.

6. Dispositif selon la revendication 4 tel que le dispositif d'introduction du flux gazeux est disposé sous le lit catalytique.

7. Dispositif selon l'une des revendications 1 à 6 tel que la zone réactionnelle est en totalité interne à la zone de distillation.

8. Dispositif selon l'une des revendications 1 à 7 tel qu'il comprend en outre un moyen de récupération de l'hydrogène en excès en tête de la zone de distillation.

9. Dispositif selon la revendication 8, tel qu'il comprend en outre un moyen de compression de l'hydrogène récupéré.

10. Dispositif selon l'une des revendications 1 à 9 tel que la partie interne de la zone réactionnelle est au moins en partie incorporée dans la zone de rectification de la zone de distillation.

11. Procédé de traitement d'une charge, constituée en majeure partie par des hydrocarbures comportant au moins 5 atomes de carbone par molécule et comprenant au moins un composé insaturé comprenant au plus six atomes de carbone par molécule dont du benzène, tel que l'on traite ladite charge dans une zone de distillation, comportant une zone d'épuisement et une zone de rectification, associée à une zone réactionnelle d'hydrogénation, au moins en partie interne à ladite zone de distillation, comprenant au moins un lit catalytique dans laquelle on réalise l'hydrogénation d'au moins une partie des composés insaturés, comprenant au plus six atomes de carbone par molécule et contenus dans la charge, en présence d'un catalyseur d'hydrogénation et d'au moins un flux gazeux comprenant de l'hydrogène, de façon à sortir finalement en tête de la zone de distillation un effluent très appauvri en composés insaturés comprenant au plus six atomes de carbone par molécule et en fond de zone de distillation un effluent appauvri en composés insaturés comprenant au plus six atomes de carbone par molécule, **caractérisé en ce que** tout lit catalytique de la partie interne de la zone d'hydrogénation est traversé à co-courant ascendant par ledit flux gazeux et le liquide et **en ce que** la vapeur de la distillation n'est pratiquement pas en contact avec le catalyseur.

12. Procédé selon la revendication 11 tel que, pour chaque lit catalytique de la partie interne de la zone réactionnelle, le liquide est distribué par un moyen de distribution du liquide situé au-dessous du lit catalytique et le flux gazeux est introduit par un moyen de distribution du flux gazeux situé au-dessous ou au sein du lit catalytique.

13. Procédé selon l'une des revendications 11 ou 12 tel que pour chaque lit catalytique de la partie interne de la zone réactionnelle, le flux gazeux introduit par un moyen d'introduction du flux gazeux dans le lit catalytique, le liquide est distribué par un moyen de distribution de liquide dans le lit catalytique, les deux moyens étant identiques.

14. Procédé selon l'une des revendications 11 ou 12 tel que le flux gazeux introduit par un moyen d'introduction du flux gazeux dans le lit catalytique, le liquide est distribué par un moyen de distribution de liquide dans le lit catalytique, le moyen d'introduction du flux gazeux étant disposé sensiblement au niveau du moyen de distribution de liquide, le gaz et le liquide étant introduits de façon séparée dans le lit catalytique.

15. Procédé selon la revendication 14 tel que le moyen d'introduction du flux gazeux est disposé au sein du lit catalytique.

16. Procédé selon la revendication 14 tel que le moyen d'introduction du flux gazeux est disposé sous lit catalytique.

17. Procédé selon l'une des revendications 11 à 16 tel que la zone d'hydrogénation est en totalité interne à la zone de distillation.

18. Procédé selon l'une des revendications 11 à 17 tel que ledit flux gazeux comprend en majeure partie de l'hydrogène.

19. Procédé selon l'une des revendications 11 à 18 tel que l'effluent de fond de la zone de distillation est mélangé à l'effluent de tête de ladite zone.

20. Procédé selon la revendication 19 tel que le mélange ainsi obtenu est, après stabilisation éventuelle, utilisé comme carburant soit directement, soit, par incorporation aux fractions carburants.

21. Procédé selon l'une des revendications 11 à 20 tel que la partie interne de la zone réactionnelle est au moins en partie incorporée dans la zone de rectification.

22. Procédé de traitement d'une charge comprenant en majeure partie des hydrocarbures oléfiniques comportant 4 atomes de carbone par molécule dont de l'isobutène, ainsi que du butène-1 et des butènes-2 en rapport correspondant sensiblement à l'équilibre thermodynamique, dans lequel on traite ladite charge dans une zone de distillation, comportant une zone d'épuisement et une zone de rectification, associée à au moins une zone réactionnelle d'hydroisomérisation, ladite zone réactionnelle d'hydroisomérisation étant au moins en partie interne à ladite zone de distillation et comprenant au moins un lit catalytique dans laquelle on réalise l'hydroisomérisation d'au moins une partie du butène-1, en présence d'un catalyseur d'hydroisomérisation et d'au moins un flux gazeux comprenant de l'hydrogène, de façon à sortir finalement en tête de la zone de distillation un effluent riche en isobutène et en fond de zone de distillation un effluent riche en butènes-2, ledit procédé étant **caractérisé en ce que** tout lit catalytique de la partie interne de la zone d'hydroisomérisation est traversé à co-courant ascendant par ledit flux gazeux et le liquide et **en ce que** la vapeur de la distillation n'est pratiquement pas en contact avec le catalyseur.

23. Procédé selon la revendication 22 tel que, pour chaque lit catalytique de la partie interne de la zone réactionnelle, le flux gazeux introduit par un moyen d'introduction du flux gazeux dans le lit catalytique, le liquide est distribué par un moyen de distribution de liquide dans le lit catalytique le moyen de distribution du liquide étant situé au-dessous du lit catalytique et le moyen de distribution du flux gazeux étant situé au-dessous ou au sein du lit catalytique.

24. Procédé selon l'une des revendications 22 et 23 tel que pour chaque lit catalytique de la partie interne de la zone réactionnelle, le flux gazeux introduit par un moyen d'introduction du flux gazeux dans le lit catalytique, le liquide est distribué par un moyen de distribution de liquide dans le lit catalytique, les deux moyens étant identiques.

25. Procédé selon l'une des revendications 22 et 23 tel que pour chaque lit catalytique de la partie interne de la zone réactionnelle, le flux gazeux introduit par un moyen d'introduction du flux gazeux dans le lit catalytique, le liquide est distribué par un moyen de distribution de liquide, le moyen d'introduction du flux gazeux étant disposé sensiblement au niveau du moyen de distribution de liquide, le gaz et le liquide étant introduits de façon séparée dans le lit catalytique.

26. Procédé selon la revendication 25 tel que le moyen d'introduction du flux gazeux est disposé au sein du lit catalytique.

27. Procédé selon la revendication 25 tel que le moyen d'introduction du flux gazeux est disposé sous lit catalytique.

28. Procédé selon l'une des revendications 22 à 28 dans lequel la zone d'hydroisomérisation est en totalité interne à la zone de distillation.

29. Procédé selon l'une des revendications 22 à 29 tel que la partie interne de la zone d'hydroisomérisation est au moins en partie dans la zone de rectification.

30. Procédé selon l'une des revendications 22 à 30 dans lequel le flux gazeux comprend en majeure partie de l'hydrogène.

## Patentansprüche

1. Vorrichtung zur reaktiven Destillation, die eine Destillationszone aufweist, die eine Erschöpfungszone und eine Rektifikationszone aufweist, die einer Reaktionszone zugeordnet ist, die wenigstens teilweise in der Destillationszone liegt und wenigstens ein Mittel zur Eindämmung wenigstens eines katalytischen Bettes umfasst, das es ermöglicht, eine Charge in Gegenwart eines Katalysators umzuwandeln, wobei die Vorrichtung wenigstens ein Mittel zur Zirkulation des größeren Teils der Destillationsflüssigkeit von unten nach oben über den Katalysator für jedes katalytische Bett des Teils der Reaktionszone innerhalb der Destillationszone umfasst; und wenigstens ein Mittel zur Zirkulation des größeren Teils des Destillationsdampfes von unten nach oben über jedes katalytische Bett des Teils der Reaktionszone innerhalb der Destillationszone umfasst, derart, dass der Destillationsdampf praktisch nicht in Kontakt mit dem Katalysator kommt; wobei die Vorrichtung **dadurch gekennzeichnet ist, dass** sie, um eine Umwandlung der Charge durch katalytische Reaktion in Gegenwart wenigstens eines gasförmigen Wasserstoff umfassenden Stromes zu ermöglichen, im übrigen ein Mittel zur Einführung des Gasstromes in die Reaktionszone und wenigstens ein Mittel zur Verteilung des größeren Teils des Gasstromes von unten nach oben über den Katalysator jedes katalytischen Bettes des Teils der Reaktionszone innerhalb der Destillationszone umfasst.

2. Vorrichtung nach Anspruch 1 derart, dass für jedes katalytische Bett des Teils innerhalb der Reaktionszone das Mittel zur Verteilung der Flüssigkeit unter dem katalytischen Bett angeordnet ist und das Mittel zur Verteilung des Gasstromes unter oder mitten in dem katalytischen Bett angeordnet ist.

3. Vorrichtung nach einem der Ansprüche 1 und 2 derart, dass die Vorrichtung zur Einführung des Gasstromes in jedes katalytische Bett identisch mit der Vorrichtung zur Verteilung der Flüssigkeit in jedem katalytischen Bett ist, das heißt, dass es ein Mittel zur Einführung des Gases in die Flüssigkeit gibt, das bezüglich der Flüssigkeitszirkulationsrichtung vor der Vorrichtung zur Flüssigkeitsverteilung liegt.

4. Vorrichtung nach einem der Ansprüche 1 und 2 derart, dass die Vorrichtung zur Einführung des Gasstromes im wesentlichen auf dem Niveau der Vorrichtung zur Flüssigkeitsverteilung angeordnet ist, wobei das Gas und die Flüssigkeit getrennt in das katalytische Bett eingeführt werden.

5. Vorrichtung nach Anspruch 4 derart, dass die Vorrichtung zur Einführung des Gasstromes mitten in dem katalytischen Bett angeordnet ist.

6. Vorrichtung nach Anspruch 4 derart, dass die Vorrichtung zur Einführung des Gasstromes unter dem katalytischen Bett angeordnet ist.

7. Vorrichtung nach einem der Ansprüche 1 bis 6 derart, dass die Reaktionszone vollständig innerhalb der Destillationszone liegt.

8. Vorrichtung nach einem der Ansprüche 1 bis 7 derart, dass sie im übrigen ein Mittel zur Gewinnung von überschüssigem Wasserstoff am Kopf der Destillationszone umfasst.

9. Vorrichtung nach Anspruch 8 derart, dass sie im übrigen ein Mittel zur Kompression des gewonnenen Wasserstoffs umfasst.

10. Vorrichtung nach einem der Ansprüche 1 bis 9 derart, dass der Teil innerhalb der Reaktionszone wenigstens teilweise in die Rektifikationszone der Destillationszone einverleibt ist.

11. Verfahren zur Behandlung einer Charge, die zum größeren Teil durch Kohlenwasserstoffe gebildet ist, die wenigstens 5 Kohlenstoffatome pro Molekül aufweisen und wenigstens eine ungesättigte Verbindung umfassen, die höchstens 6 Kohlenstoffatome pro Molekül, darunter Benzol umfasst, derart, dass man die Charge in einer Destillationszone behandelt, die eine Erschöpfungszone und eine Rektifikationszone umfasst, die einer Hydrierreaktionszone zugeordnet ist, die wenigstens teilweise in der Destillationszone liegt und wenigstens ein katalytisches Bett umfasst, in dem man die Hydrierung wenigstens eines Teils der ungesättigten Verbindungen, die höchstens sechs Kohlenstoffatome pro Molekül umfassen und in der Charge enthalten sind, in Gegenwart eines Hydrierungskatalysators und wenigstens eines gasförmigen wasserstoffhaltigen Stromes derart verwirklicht, dass letztendlich am Kopf der Destillationszone ein an ungesättigten Verbindungen, die höchstens 6 Kohlenstoffatome pro Molekül umfassen, sehr verarmter Abstrom und am Boden der Destillationszone ein an ungesättigten Verbindungen, die höchstens 6 Kohlenstoffatome pro Molekül umfassen, verarmter Abstrom austritt, **dadurch gekennzeichnet, dass** jedes katalytische Bett des Teils innerhalb der Hydrierungszone im Gleichstrom aufsteigend durch den Gas- und Flüssigstrom durchsetzt wird und dass der Destillationsdampf praktisch nicht in Kontakt mit dem Katalysator kommt.

12. Verfahren nach Anspruch 11 derart, dass für jedes katalytische Bett des Teils innerhalb der Reaktionszone die Flüssigkeit durch ein Mittel zur Verteilung der Flüssigkeit verteilt wird, das unter dem katalytischen Bett angeordnet ist und der Gasstrom durch ein Mittel zur Verteilung des Gasstroms eingeführt wird, das unter oder innerhalb des katalytischen Bettes angeordnet ist.

13. Verfahren nach einem der Ansprüche 11 oder 12, derart, dass für jedes katalytische Bett des Teils innerhalb der Reaktionszone bei Einführen des Gasstroms durch ein Mittel zur Einführung des Gasstroms in das katalytische Bett, die Flüssigkeit durch ein Mittel zur Verteilung der Flüssigkeit in dem katalytischen Bett verteilt wird, wobei die beiden Mittel identisch sind.

14. Verfahren nach einem der Ansprüche 11 oder 12, derart, dass bei Einführen des Gasstroms durch ein Mittel zur Einführung des Gasstroms in das katalytische Bett, die Flüssigkeit durch ein Mittel zur Verteilung der Flüssigkeit in dem katalytischen Bett verteilt wird, wobei das Mittel zur Einführung des Gasstroms im wesentlichen auf dem Niveau des Mittels zur Flüssigkeitsverteilung angeordnet ist, wobei das Gas und die Flüssigkeit getrennt in das katalytische Bett eingeführt werden.

15. Verfahren nach Anspruch 14 derart, dass das Mittel zur Einführung des Gasstroms in dem katalytischen Bett angeordnet ist.

16. Verfahren nach Anspruch 14 derart, dass das Mittel zur Einführung des Gasstroms unter dem katalytischen Bett angeordnet ist.

17. Verfahren nach einem der Ansprüche 11 bis 16, derart, dass die Hydrierungszone vollständig in der Destillationszone liegt.

18. Verfahren nach einem der Ansprüche 11 bis 17 derart, dass der Gasstrom den größeren Teil des Wasserstoffs umfasst.

19. Verfahren nach einem der Ansprüche 11 bis 18 derart, dass der Bodenabstrom der Destillationszone zum Kopfabstrom dieser Zone gemischt wird.

20. Verfahren nach Anspruch 19 derart, dass das so erhaltene Gemisch nach eventueller Stabilisierung entweder als Treibstoff direkt oder durch Einverleiben in die anderen Treibstofffraktionen verwendet wird.

21. Verfahren nach einem der Ansprüche 11 bis 20 derart, dass der Teil in der Reaktionszone wenigstens teilweise in die Rektifikationszone der Destillationszone einverleibt ist.

22. Verfahren zur Behandlung einer Charge, die zum größeren Teil olefinische Kohlenwasserstoffe, die 4 Kohlenstoffatome pro Molekül umfassen, darunter Isobuten sowie 1-Buten und 2-Butene in einem Verhältnis umfassen, das im wesentlichen dem thermodynamischen Gleichgewicht entspricht, in welchem Verfahren man die Charge in einer Destillationszone behandelt, die eine Erschöpfungszone und eine Rektifikationszone umfasst, die einer Hydrierisomerisierungszone zugeordnet ist, wobei die Reaktionszone wenigstens teilweise in der Destillationszone angeordnet ist und wenigstens ein katalytisches Bett umfasst, in dem man die Hydrierisomerisierung wenigstens eines Teils des 1-Butens in Gegenwart eines Hydrierisomerisierungskatalysators und wenigstens eines gasförmigen wasserstoffhaltigen Stromes derart verwirklicht, dass letztendlich am Kopf der Destillationszone ein an Isobuten reicher Abstrom und am Boden der Destillationszone ein an 2-Butenen reicher Abstrom austritt, wobei das Verfahren **dadurch gekennzeichnet ist, dass** jedes katalytische Bett des Teils innerhalb der Hydrierisomerisierungszone im aufsteigenden Gleichstrom durch den Gas- und Flüssigstrom durchsetzt wird und dadurch, dass der Destillationsdampf praktisch nicht in Kontakt mit dem Katalysator kommt.

23. Verfahren nach Anspruch 22 derart, dass für jedes katalytische Bett des Teils innerhalb der Reaktionszone bei Einführen des Gasstroms durch ein Mittel zur Einführung des Gasstroms in das katalytische Bett, die Flüssigkeit durch ein Mittel zur Verteilung der Flüssigkeit in dem katalytischen Bett verteilt wird, das unter dem katalytischen Bett angeordnet ist und wobei das Mittel zur Verteilung des Gasstroms unterhalb oder innerhalb des katalytischen Bettes angeordnet ist.

24. Verfahren nach einem der Ansprüche 22 und 23 derart, dass für jedes katalytische Bett des Teils innerhalb der Reaktionszone bei Einführen des Gasstroms durch ein Mittel zur Einführung des Gasstroms in das katalytische Bett, die Flüssigkeit durch ein Mittel zur Verteilung der Flüssigkeit in dem katalytischen Bett verteilt wird, wobei die beiden Mittel identisch sind.

25. Verfahren nach einem der Ansprüche 22 und 23 derart, dass für jedes katalytische Bett des Teils innerhalb der Reaktionszone bei Einführen des Gasstroms durch ein Mittel zur Einführung des Gasstroms in das katalytische Bett, die Flüssigkeit durch ein Mittel zur Verteilung der Flüssigkeit verteilt wird, wobei das Mittel zur Einführung des Gasstroms im wesentlichen auf dem Niveau des Mittels zur Flüssigkeitsverteilung angeordnet ist, wobei das Gas und die Flüssigkeit getrennt in das katalytische Bett eingeführt werden.

26. Verfahren nach Anspruch 25 derart, dass das Mittel zur Einführung des Gasstroms in dem katalytischen Bett angeordnet ist.

27. Verfahren nach Anspruch 25 derart, dass das Mittel zur Einführung des Gasstroms unter dem katalytischen Bett angeordnet ist.

28. Verfahren nach einem der Ansprüche 22 bis 27 derart, dass die Hydrierisomerisierungszone vollständig in der Destillationszone liegt.

29. Verfahren nach einem der Ansprüche 22 bis 28 derart, dass der Teil innerhalb der Hydrierisomerisierungszone wenigstens teilweise in der Rektifikationszone liegt.

30. Verfahren nach einem der Ansprüche 22 bis 29, bei dem der Gasstrom den größeren Teil des Wasserstoffs umfasst.

## Claims

1. A reactive distillation apparatus comprising a distillation zone which comprises a stripping zone and a rectification zone associated with a reaction zone, at least a portion of which is internal to said distillation zone, and comprising at least one means for withholding at least one catalyst bed, which makes it possible to convert a feed in the presence of a catalyst, said apparatus comprising at least one means for circulating the major portion of the liquid from the bottom to the top through the catalyst for each catalyst bed in the portion of said reaction zone which is internal to said distillation zone; and at least one means for circulating the major portion of the distillation vapour from the bottom to the top through each catalyst bed of the portion of the reaction zone which is internal to said distillation zone, in such a manner that said distillation vapour is substantially not in contact with the catalyst; said apparatus being **characterized in that**, in order to make it possible to carry out a conversion of said feed by catalytic reaction in the presence of at least one gas stream comprising hydrogen, it further comprises at least one means for introducing said gas stream into said reaction zone and at least one means for distributing of the major portion of said gas stream from the bottom to the top through each catalyst bed of the portion of the reaction zone which is internal to said distillation zone.

2. An apparatus according to claim 1 in which, for each catalyst bed in the internal portion of the reaction zone, the liquid distribution means is located below the catalyst bed and the gas stream distribution means is located below or within the catalyst bed.

3. An apparatus according to any one of claims 1 and 2, in which the apparatus for introducing the gas stream into each catalyst bed is identical to the apparatus for distributing liquid in the catalyst bed, i.e., there is a means for introducing the gas into the liquid that is located upstream of the liquid distribution apparatus with respect to the direction of liquid circulation.

4. An apparatus according to any one of claims 1 and 2, in which the gas stream introduction apparatus is located substantially at the level of the liquid distribution apparatus, the gas and the liquid being introduced separately into the catalyst bed.

5. An apparatus according to claim 4, in which the gas stream introduction apparatus is located within the catalyst bed.

6. An apparatus according to claim 4, in which the gas stream introduction apparatus is located below the catalyst bed.

7. An apparatus according to any one of claims 1 to 6, in which the reaction zone is totally internal to the distillation zone.

8. An apparatus according to any one of claims 1 to 7, which further comprises a means for recovering excess hydrogen at the head of the distillation zone.

9. An apparatus according to claim 8, which further comprises a means for compressing the recovered hydrogen.

10. An apparatus according to any one of claims 1 to 9 in which the internal portion of the reaction zone is at least partially incorporated in the rectification zone.

11. A process for the treatment of a feed, a major portion of which is constituted by hydrocarbons containing at least 5 carbon atoms per molecule and comprising at least one unsaturated compound containing at most six carbon atoms per molecule including benzene, in which said feed is treated in a distillation zone comprising a stripping zone and a rectification zone, associated with a hydrogenation reaction zone which is at least partially internal to said distillation zone, comprising at least one catalyst bed, in which at least a portion of the unsaturated compounds containing at most six carbon atoms per molecule and contained in the feed are hydrogenated in the presence of a hydrogenation catalyst and at least one gas stream comprising hydrogen so as to cause an effluent which is highly depleted in unsaturated compounds containing at most six carbon atoms per molecule to leave overhead of the distillation zone and an effluent which is depleted in unsaturated compounds containing at most six carbon atoms per molecule to leave from the bottom of the distillation zone, **characterized in that** each catalyst bed of the internal portion of the hydrogenation zone is traversed by an ascending co-current of said gas stream and liquid, and **in that** the vapour from the distillation zone does not substantially come into contact with the catalyst.

12. A process according to claim 11, in which, for each catalyst bed of the portion internal to the reaction zone, the liquid is distributed by a liquid distribution means located above the catalyst bed and the gas stream is introduced by a distribution means located below or within the catalyst bed.

13. A process according to any one of claims 11 and 12, in which, for each catalyst bed of the portion internal to the reaction zone, the gas stream is introduced by a gas stream introduction means into the catalyst bed, the liquid is distributed by a liquid distribution means into the catalyst bed and both means are identical.

14. A process according to any one of claims 11 and 12, in which the gas stream is introduced by a gas stream introduction means into the catalyst bed, the liquid is distributed by a liquid distribution means into the catalyst bed, the means for introducing the gas stream is substantially at the level of the means for distributing the liquid and the gas and the liquid are introduced separately into the catalyst bed.

15. A process according to claim 14, in which the gas stream introduction means is located within the catalyst bed.

16. A process according to claim 14, in which the gas stream introduction means is located below the catalyst bed.

17. A process according to any one of claims 11 to 16, in which the hydrogenation zone is totally internal to the distillation zone.

18. A process according to any one of claims 11 to 17, in which the major portion of said gas stream is hydrogen.

19. A process according to any one of claims 11 to 18, in which the bottom effluent from the distillation zone is mixed with the overhead effluent from said zone.

20. A process according to claim 19, in which the mixture obtained, after any required stabilisation, is used as a fuel either directly or by incorporation into fuel fractions.

21. A process according to any one of claims 11 to 20, in which the internal portion of the reaction zone is at least partially incorporated in the rectification zone.

22. A process for the treatment of a feed comprising, as its major portion, olefinic hydrocarbons containing 4 carbon atoms per molecule, including isobutene, also 1-butene and 2-butenes in a ratio which substantially corresponds to the thermodynamic equilibrium, in which said feed is treated in a distillation zone comprising a stripping zone and a rectification zone associated with at least one hydroisomerisation reaction zone, said hydroisomerisation reaction zone being at least partially internal to said distillation zone and comprising at least one catalyst bed, in which hydroisomerisation of at least a portion of 1-butene is carried out in the presence of a hydroisomerisation catalyst and at least one gas stream comprising hydrogen, such that an effluent which is rich in isobutene leaves the distillation zone overhead and an effluent which is rich in 2-butenes leaves the bottom, said process being **characterized in that** each catalyst bed in the internal portion of the hydroisomerisation zone is traversed by an ascending co-current of said gas stream and liquid and the distillation vapour is substantially not in contact with the catalyst.

23. A process according to claim 22, in which, for each catalyst bed of the portion internal to the reaction zone, the gas stream is introduced by a gas stream introduction means into the catalyst bed, the liquid is distributed by a liquid distribution means into the catalyst bed, the liquid distribution means is located below the catalyst bed and the gas stream distribution means is located below or within the catalyst bed.

24. A process according to any one of claims 22 and 23, in which, for each catalyst bed of the portion internal to the reaction zone, the gas stream is introduced by a gas stream introduction means into the catalyst bed, the liquid is distributed by a liquid distribution means into the catalyst bed and both means are identical.

25. A process according to any one of claims 22 and 23, in which, for each catalyst bed in the internal portion of the reaction zone, the gas stream is introduced by a gas stream introduction means into the catalyst bed, the liquid is distributed by a liquid distribution means, the means for introducing the gas stream is located substantially at the level of the means for distributing the liquid and the gas and the liquid are introduced separately into the catalyst bed.

26. A process according to claim 25, in which the gas stream introduction means is located within the catalyst bed.

27. A process according to claim 25, in which the gas stream introduction means is located below the catalyst bed.

28. A process according to any one of claims 22 to 28, in which the internal portion of the hydroisomerisation zone is totally internal to the distillation zone.

29. A process according to any one of claims 22 to 29, in which the internal portion of the hydroisomerisation zone is at least partially in the rectification zone.

30. A process according to claims 22 to 30, in which a major portion of the gas stream is hydrogen.
